(19) Europäisches Patentamt / European Patent Office / Office européen des brevets

(11) EP 2 251 052 A1

(12) **EUROPEAN PATENT APPLICATION**
published in accordance with Art. 153(4) EPC

(43) Date of publication:
**17.11.2010 Bulletin 2010/46**

(21) Application number: **09708712.6**

(22) Date of filing: **06.02.2009**

(51) Int Cl.:
***A61M 1/14*** *(2006.01)*

(86) International application number:
**PCT/JP2009/000480**

(87) International publication number:
**WO 2009/098897 (13.08.2009 Gazette 2009/33)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **07.02.2008 JP 2008028043**

(71) Applicant: **JMS Co., Ltd.**
**Hiroshima-shi**
**Hiroshima 730-8652 (JP)**

(72) Inventor: **IKEDA, Atsushi**
**Hiroshima-shi**
**Hiroshima 730-8652 (JP)**

(74) Representative: **Collin, Jérôme et al**
**Cabinet Régimbeau**
**20, rue de Chazelles**
**75847 Paris Cedex 17 (FR)**

(54) **BLOOD DIALYZER**

(57) A hemodialysis apparatus includes a hemodialysis treatment administering unit including a blood treating unit for treating patient's blood; and a controller for controlling the hemodialysis treatment administering unit. The controller includes a maximum change rate setting unit for setting the maximum rate of change in circulating blood volume, and an adjustment line forming unit for forming an adjustment line for adjusting the rate of change in circulating blood volume so as to be equal to or less than the maximum change rate set in the maximum change rate setting unit. The controller controls the hemodialysis treatment administering unit based on the adjustment line formed in the adjustment line forming unit.

FIG. 5

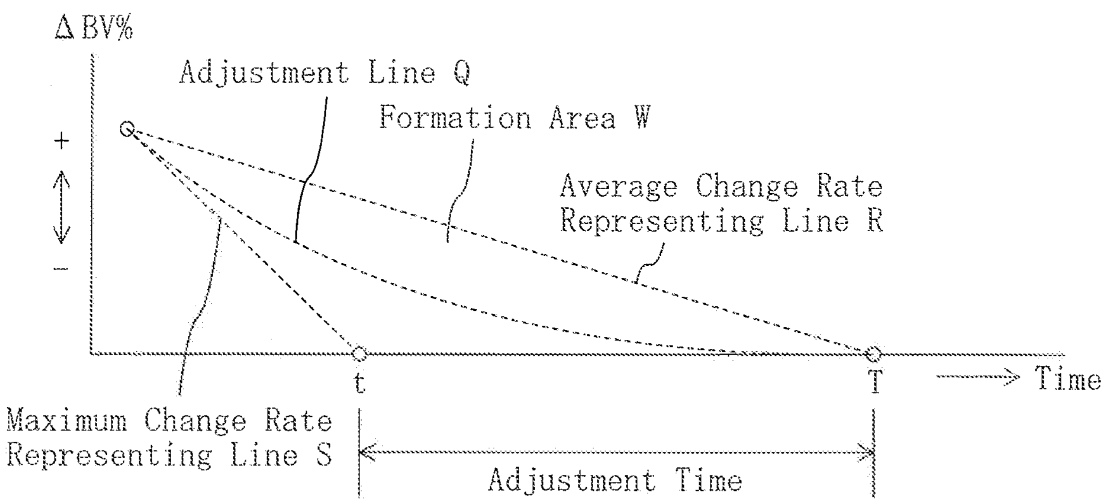


EP 2 251 052 A1

Printed by Jouve, 75001 PARIS (FR)

## Description

TECHNICAL FIELD

The present invention relates to a hemodialysis apparatus for performing a predetermined treatment by extracorporeally circulating patient's blood.

BACKGROUND ART

Conventionally, hemodialysis treatment using a hemodialysis apparatus has been provided for patients experiencing renal failure. The hemodialysis apparatus includes a blood circuit through which blood is obtained from and returned to a patient; a dialyzer provided in the middle of the blood circuit; and a dialysate circuit for supplying dialysate to the dialyzer. The blood and dialysate circuits are controlled by a control unit, and extracorporeal circulation is performed, in which blood obtained from a patient flows into the dialyzer to be returned to the patient. During such extracorporeal circulation, the blood is purified by dialysate supplied to the dialyzer, and body fluid in the blood is removed.
When providing the hemodialysis treatment by using the above-described hemodialysis apparatus, a medical staff sets a hemodialysis time, a dialysate flow rate, the volume of body fluid to be removed, etc. depending on patient's conditions.

CITATION LIST

PATENT DOCUMENT

PATENT DOCUMENT 1: Japanese Patent Publication No. 2007-130300

SUMMARY OF THE INVENTION

TECHNICAL PROBLEM

A patient's circulating blood volume at an initial stage of the hemodialysis treatment exceeds a target circulating blood volume, and the body fluid removal allows the patient's circulating blood volume to approach an appropriate circulating blood volume. At such a stage, if the rate of change in circulating blood volume is too fast, or the circulating blood volume is excessively decreased, a decrease in blood pressure is caused.
In addition, there may be a method for removing body fluid while maintaining the constant rate of change in circulating blood volume, or the constant circulating blood volume. However, such a method causes a decrease in plasma refilling rate (PRR) and in the rate of body fluid removal, resulting in an increase in hemodialysis time.
The rate of change in circulating blood volume may be set in advance so as to avoid the above-described situations, thereby adjusting the actual rate of change in circulating blood volume. However, it is particularly difficult for an inexperienced medical staff to learn how to adjust such a rate. In addition, if the rate of change in circulating blood volume is inappropriately adjusted by a patient, there are possibilities that a risk to the patient is increased, and that the hemodialysis time is unnecessarily extended, resulting in an inefficient hemodialysis treatment.
The present invention has been made in view of the foregoing, and it is an object of the present invention to easily adjust the rate of change in circulating blood volume so as to approach an appropriate value even by an inexperienced medical staff, thereby reducing the risk to a patient during the hemodialysis treatment to improve safety, and efficiently performing the hemodialysis treatment.

SOLUTION TO THE PROBLEM

In the present invention, in order to accomplish the above-described object, the maximum rate of change in circulating blood volume, and the rate of change in circulating blood volume is adjusted so as to be equal to or less than such maximum change rate.
Specifically, in a first aspect of the invention, a hemodialysis apparatus performing a predetermined treatment by extracorporeally circulating patient's blood includes a hemodialysis treatment administering unit including a blood treating unit for treating patient's blood; and a controller for controlling the hemodialysis treatment administering unit. The controller includes a maximum change rate setting unit for setting the maximum rate of change in circulating blood volume, and an adjustment line forming unit for forming an adjustment line for adjusting the rate of change in circulating blood volume so as to be equal to or less than the maximum change rate set in the maximum change rate setting unit; and controls the hemodialysis treatment administering unit based on the adjustment line formed in the adjustment line forming unit.

According to the foregoing configuration, the maximum change rate setting unit of the controller sets the maximum rate of change in circulating blood volume. The maximum change rate is obtained depending on patient's conditions in advance, and can be set regardless of medical staffs experiences. The adjustment line formed in the adjustment line forming unit is for adjusting the rate of change in circulating blood volume so as to be equal to or less than the maximum change rate. The hemodialysis treatment administering unit is controlled based on the adjustment line, thereby not performing the hemodialysis treatment with the high rate of change in circulating blood volume, which is unsuitable to a patient. Further, the adjustment line is not inappropriately formed even by an inexperienced medical staff.

A second aspect of the invention is intended for the hemodialysis apparatus of the first aspect of the invention, in which the controller displays the adjustment line on an indicator of the controller.

According to the foregoing configuration, the shape of the adjustment line can be visually viewed.

A third aspect of the invention is intended for the hemodialysis apparatus of the second aspect of the invention, in which the controller displays a maximum change rate representing line which represents the maximum change rate, on the indicator.

According to the foregoing configuration, a relationship between the maximum change rate and the adjustment line can be viewed.

A fourth aspect of the invention is intended for the hemodialysis apparatus of any one of the first to third aspects of the invention, in which the controller includes a storage unit for storing a plurality of options of the maximum change rate; and in which the maximum change rate setting unit sets one of the plurality of options of the maximum change rate, which are stored in the storage unit.

According to the foregoing configuration, when setting the maximum rate of change in circulating blood volume, any one of the plurality of options of the maximum change rate, which are stored in the storage unit, is simply selected. Thus, the maximum change rate can be easily set.

A fifth aspect of the invention is intended for the hemodialysis apparatus of any one of the first to fourth aspects of the invention, in which the controller includes an average change rate setting unit for setting the average rate of change in circulating blood volume within a total hemodialysis time; and in which the adjustment line forming unit forms the adjustment line between an average change rate representing line which represents the average change rate set in the average change rate setting unit, and the maximum change rate representing line.

According to the foregoing configuration, the shape of the adjustment line is defined considering the average change rate.

A sixth aspect of the invention is intended for the hemodialysis apparatus of the fifth aspect of the invention, in which the average change rate setting unit sets the average rate of change in circulating blood volume within the hemodialysis time so that a circulating blood volume when starting hemodialysis treatment reaches a target circulating blood volume at a preset termination point of the hemodialysis treatment.

According to the foregoing configuration, the adjustment line can be formed considering the average change rate which reaches the target circulating blood volume at the preset termination point of the hemodialysis treatment. This allows the circulating blood volume to reach the target circulating blood volume at the termination point of the hemodialysis treatment, thereby avoiding extension of the hemodialysis time.

A seventh aspect of the invention is intended for the hemodialysis apparatus of the any one of the first to sixth aspects of the invention, in which the adjustment line forming unit arranges a plurality of adjustment line forming blocks in a direction representing a lapse of the hemodialysis time; and arranges at least one of the adjustment line forming blocks so as to be apart from the adjacent adjustment line forming block in the direction representing the lapse of the hemodialysis time in order to form the adjustment line extending so as to connect the adjacent adjustment line forming blocks.

According to the foregoing configuration, the adjacent adjustment line forming blocks are apart from each other in the direction representing the elapse of the hemodialysis time, thereby reducing an slope angle of the adjustment line extending so as to connect the adjacent adjustment line forming blocks. That is, a clearance size between the adjacent adjustment line forming blocks is changed, thereby changing the rate of change in circulating blood volume.

An eighth aspect of the invention is intended for the hemodialysis apparatus of the seventh aspect of the invention, in which a plurality of options of data associated with the number of adjustment line forming blocks are stored in the controller; and in which any data option can be selected from the options of the data associated with the number of adjustment line forming blocks.

According to the foregoing configuration, the number of adjustment line forming blocks can be easily set.

A ninth aspect of the invention is intended for the hemodialysis apparatus of the seventh or eighth aspect of the invention, in which time adjustment block(s) arc arranged between the adjacent adjustment line forming blocks.

According to the foregoing configuration, the clearance size between the adjacent adjustment line forming blocks can be easily changed depending on the number of time adjustment blocks.

A tenth aspect of the invention is intended for the hemodialysis apparatus of the ninth aspect of the invention, in which the adjustment line forming unit can change a pattern relating to the number of time adjustment blocks to be arranged.

According to the foregoing configuration, the pattern relating to the number of time adjustment blocks to be arranged is changed, thereby changing the clearance size between the adjacent adjustment line forming blocks.

An eleventh aspect of the invention is intended for the hemodialysis apparatus of the tenth aspect of the invention, in which the adjustment line forming unit displays a plurality of patterns relating to the number of time adjustment blocks to be arranged, on the indicator of the controller; and can select one of the displayed patterns.

According to the foregoing configuration, any pattern can be selected while displaying and viewing the plurality of patterns relating to the number of time adjustment blocks to be arranged on the indicator.

A twelfth aspect of the invention is intended for the hemodialysis apparatus of the tenth or eleventh aspect of the invention, in which the pattern relating to the number of time adjustment blocks to be arranged is a pattern according to a predetermined sequence.

According to the foregoing configuration, the arrangement of and the number of time adjustment blocks can be easily set by using the sequence.

A thirteenth aspect of the invention is intended for the hemodialysis apparatus of any one of the ninth to twelfth aspects of the invention, in which the time adjustment block adjacent to the adjustment line forming block positioned at the end in the direction representing the lapse of the hemodialysis time is formed so as to absorb temporal errors caused due to arrangement of other time adjustment blocks.

According to the foregoing configuration, the temporal errors caused when arranging a plurality of time adjustment blocks to form the adjustment line can be absorbed.

A fourteenth aspect of the invention is intended for the hemodialysis apparatus of any one of the seventh to thirteenth aspects of the invention, in which the adjustment line forming unit can change a shape of the adjustment line by shifting any adjustment line forming block after formation of the adjustment line.

According to the foregoing configuration, the shape of the adjustment line which has been formed once can be changed by shifting the adjustment line forming block.

A fifteenth aspect of the invention is intended for the hemodialysis apparatus of the fourteenth aspect of the invention, in which the adjustment line forming unit changes the hemodialysis time by shifting the adjustment line forming block corresponding to an end point of the hemodialysis time; and adjusts clearances between the adjacent adjustment line forming blocks so that the pre-change hemodialysis time becomes coincident with the changed hemodialysis time.

According to the foregoing configuration, when a medical staff changes the hemodialysis time, the adjustment line corresponding to the changed hemodialysis time can be formed without an operation for adjusting the clearances between the adjustment line forming blocks.

A sixteenth aspect of the invention is intended for the hemodialysis apparatus of the fourteenth or fifteenth aspect of the invention, in which a shape of the adjustment line forming block is changed.

A seventeenth aspect of the invention is intended for the hemodialysis apparatus of any one of the fourteenth to sixteenth aspects of the invention, in which a shape of the time adjustment block is changed.

An eighteenth aspect of the invention is intended for the hemodialysis apparatus of any one of the fourteenth to sixteenth aspects of the invention, in which the adjustment line forming unit can shift only the adjustment line forming blocks subsequent to a point at which the hemodialysis time has elapsed, during the hemodialysis treatment.

According to the foregoing configuration, if the shape of the adjustment line is necessary to be changed during the hemodialysis treatment, only the adjustment line forming blocks subsequent to the point at which the hemodialysis time has elapsed can be shifted, thereby remaining the shape of the adjustment line up to a point at which the hemodialysis treatment has been performed.

A nineteenth aspect of the invention is intended for the hemodialysis apparatus of any one of the fourteenth to eighteenth aspects of the invention, in which the adjustment line forming unit can change the shape of the adjustment line by parallel-shifting a portion of the adjustment line subsequent to the point at which the hemodialysis time has elapsed with its shape being maintained.

A twentieth aspect of the invention is intended for the hemodialysis apparatus of any one of the seventh to nineteenth aspects of the invention, in which the adjustment line forming unit displays the adjustment line forming blocks on the indicator of the controller.

According to the foregoing configuration, the adjustment line forming block can be shifted while viewing it on the indicator.

## ADVANTAGES OF THE INVENTION

According to the first aspect of the invention, the maximum rate of change in circulating blood volume is set, and then the adjustment line for adjusting the rate of change in circulating blood volume so as to be equal to less than the maximum change rate is formed. Subsequently, the hemodialysis treatment administering unit is controlled based on the adjustment line. Thus, the rate of change in circulating blood volume is not increased to such an extent as to be suitable to a patient, and the appropriate adjustment line can be easily formed even by an inexperienced medical staff. This reduces a risk to a patient during the hemodialysis treatment to improve safety, and allows an efficient hemodialysis treatment.

According to the second aspect of the invention, the adjustment line is displayed on the indicator. Thus, a medical staff can be visually view whether or not the adjustment line is correctly formed, thereby further improving the safety of a patient.

According to the third aspect of the invention, the maximum change rate representing line is displayed on the indicator together with the adjustment line, thereby more easily viewing whether or not the adjustment line is correctly formed.

According to the fourth aspect of the invention, the plurality of options of the maximum change rate are stored in the storage unit, and any one of the plurality of options of the maximum change rate is simply selected to set the maximum change rate. Thus, the maximum change rate suitable to patient's conditions can be easily set.

According to the fifth aspect of the invention, the average rate of change in circulating blood volume within the hemodialysis time can be set to form the adjustment line between the average change rate representing line and the maximum change rate representing line. Thus, the adjustment line can be formed in a more suitable shape.

According to the sixth aspect of the invention, the average rate of change in circulating blood volume can be set so that the circulating blood volume when starting the hemodialysis treatment reaches the target circulating blood volume at the preset termination point of the hemodialysis treatment. In addition, the adjustment line is formed between the average change rate representing line which represents the average change rate, and the maximum change rate representing line, thereby reducing an unnecessary extension of the hemodialysis time.

According to the seventh aspect of the invention, the plurality of adjustment line forming blocks arranged in the direction representing the lapse of the hemodialysis time are arranged so as to be apart from each other, and the adjustment line extending so as to connect the adjacent adjustment line forming blocks is formed. Thus, the rate of change in circulating blood volume can be arbitrarily changed depending of the clearance size between the adjustment, line forming blocks.

According to the eighth aspect of the invention, the plurality of options of data associated with the number of adjustment line forming blocks are stored in the controller, and any data option can be selected from such data options. Thus, the number of adjustment line forming blocks can be easily set, thereby improving ease of use.

According to the ninth aspect of the invention, the time adjustment block(s) are arranged between the adjacent adjustment line forming blocks. Thus, the number of time adjustment blocks to be arranged between the adjustment line forming blocks is simply changed to change the clearance size between the adjacent adjustment line forming blocks, thereby easily changing the rate of change in circulating blood volume.

According to the tenth aspect of the invention, the pattern relating to the number of time adjustment blocks to be arranged between the adjacent adjustment line forming blocks is simply changed, thereby easily changing the rate of change in circulating blood volume so as to correspond to such a pattern.

According to the eleventh aspect of the invention, any pattern can be selected with the plurality of patterns relating to the number of time adjustment blocks to be arranged being displayed on the indicator. Thus, the ease of use when selecting the pattern relating to the number of time adjustment blocks to be arranged can be improved.

According to the twelfth aspect of the invention, the pattern relating to the number of time adjustment blocks to be arranged is the pattern according to the predetermined sequence. Thus, the arrangement of and the number of time adjustment blocks can be easily set.

According to the thirteenth aspect of the invention, when arranging the plurality of time adjustment blocks, the time adjustment block adjacent to the adjustment line forming block positioned at the end in the direction representing the lapse of the hemodialysis time can absorb the temporal errors.

According to the fourteenth aspect of the invention, any adjustment line forming block can be shifted after the formation of the adjustment line, thereby correcting the shape of the adjustment line which has been formed once, so as to be suitable to patient's conditions. Thus, the adjustment line can be more suitably formed.

According to the fifteenth aspect of the invention, after the hemodialysis time is changed, the clearances between the adjustment line forming blocks are adjusted so that the pre-change hemodialysis time becomes coincident with the changed hemodialysis time. Thus, when a medical staff changes the hemodialysis time, the adjustment line corresponding to the changed hemodialysis time can be formed without the operation for adjusting the clearances between the adjustment line forming blocks. Consequently, the ease of use can be improved.

According to the sixteenth aspect of the invention, the shape of the adjustment line forming block is changed, thereby changing the shape, of the adjustment line.

According to the seventeenth aspect of the invention, the shape of the time adjustment block is changed, thereby changing the shape of the adjustment line.

According to the eighteenth aspect of the invention, only the final adjustment line forming block subsequent to the point at which the hemodialysis time has elapsed can be shifted during the hemodialysis treatment. Thus, the shape of the adjustment line up to the point at which the hemodialysis treatment has been performed can be remained, and the shape of the subsequent portion of the adjustment line can be set based on such a shave.

According to the nineteenth aspect of the invention, when changing the shape of the adjustment line, the shape of the adjustment line before the shape change can be used.

According to the twentieth aspect of the invention, the adjustment line forming blocks are displayed on the indicator, thereby improving the ease of use when shifting the adjustment line forming block.

## BRIEF DESCRIPTION OF THE DRAWINGS

[FIG. 1] FIG. 1 is a view illustrating a usage state of a hemodialysis apparatus of the present invention.

[FIG. 2] FIG. 2 is a block diagram of the hemodialysis apparatus.

[FIG. 3] FIG. 3 is a view illustrating a display panel on which a stop mode window is displayed.

[FIG. 4] FIG. 4 is a view illustrating the display panel on which selection buttons for selecting the maximum rate of change in circulating blood volume are displayed.

[FIG. 5] FIG. 5 is a graph illustrating the maximum change rate, an average change rate, and an adjustment line.

[FIG. 6] FIG. 6 is a conceptual illustration of adjustment line forming blocks.

[FIG. 7] FIG. 7 is an enlarged view of the adjustment line forming block.

[FIG. 8] FIG. 8 is a view illustrating the adjustment line forming blocks and time adjustment blocks which are arranged to form an adjustment line.

[FIG. 9] FIG. 9 is an enlarged view of the time adjustment block.

[FIG. 10] FIG. 10 is a view illustrating the display panel on which selection buttons for selecting a pattern relating to the number of time adjustment blocks to be arranged are displayed.

[FIG. 11] FIG. 11 is a graph illustrating an adjustment line when the pattern relating to the number of time adjustment blocks to be arranged is an A pattern.

[FIG. 12] FIG. 12. is a conceptual illustration of the adjustment line formed in the A pattern.

[FIG. 13] FIG. 13 is a graph illustrating an adjustment line when the pattern relating to the number of time adjustment blocks to be arranged is a B pattern.

[FIG. 14] FIG. 14 is a graph illustrating an adjustment line when the pattern relating to the number of time adjustment blocks to be arranged is a C pattern.

[FIG. 15] FIG. 15 is a graph illustrating an adjustment line when the pattern relating to the number of time adjustment blocks to be arranged is a D pattern.

[FIG. 16] FIG. 16 is a graph illustrating an adjustment line when the pattern relating to the number of time adjustment blocks to be arranged is an E pattern.

[FIG. 17] FIG. 17 is a graph illustrating an adjustment line when the pattern relating to the number of time adjustment blocks to be arranged is an F pattern.

[FIG. 18] FIG. 18 is a view illustrating the display panel on which a maximum change rate representing line, an average change rate representing line, and an adjustment line.

[FIG. 19] FIGS. 19 are equivalent to FIG. 18. FIG. 19(a) is a graph illustrating a case where a circulating blood volume change ratio is decreased. FIG. 19(b) is a graph illustrating a case where the circulating blood volume change ratio is increased.

[FIG. 20] FIGS. 20 are equivalent to FIG. 18. FIG. 20(a) is a graph illustrating a case where a hemodialysis time is shortened. FIG. 20(b) is a graph illustrating a case where the hemodialysis time is extended.

[FIG. 21] FIG. 21 is equivalent to FIG. 18, and is a graph illustrating a case where a start point of a BV navigation function is changed.

[FIG. 22] FIG. 22 is equivalent to FIG. 18, and is a graph illustrating a case where a first half of the BV navigation is changed.

[FIG. 23] FIG. 23 is equivalent to FIG. 18, and is a graph illustrating a case where a second half of the BV navigation is changed.

[FIG. 24] FIG. 24 is a graph illustrating a case where a shape of the adjustment line by shifting the adjustment line forming block.

[FIG. 25] FIG. 25 is a graph illustrating a case where the hemodialysis time is extended by shifting the final adjustment line forming block after hemodialysis treatment is started.

[FIG. 26] FIG. 26 is a graph illustrating a case where the circulating blood volume change ratio is increased by shifting the final adjustment line forming block after the hemodialysis treatment is started.

[FIG. 27] FIG. 27 is a graph illustrating a case where the shape of the adjustment line is changed by shifting the middle adjustment line forming block.

DESCRIPTION OF REFERENCE CHARACTERS

| | |
|---|---|
| 1 | Hemodialysis Apparatus |
| 2 | Hemodialysis Treatment Administering Unit |
| 3 | Operation Display Unit |
| 4 | Controller |
| 5 | Auxiliary Memory (Storage Unit) |
| 10 | Dialyzer (Blood Treating Unit) |
| 29 | Display Panel (Indicator) |
| 31 | Maximum Change Rate Setting Unit |
| 32 | Average Change Rate Setting Unit |
| 33 | Adjustment Line Forming Unit |
| S | Maximum Change Rate Representing Line |
| R | Average Change Rate Representing Line |
| Q | Adjustment Line |
| W | Formation Area |

DESCRIPTION OF EMBODIMENT

An embodiment of the present invention will be described in detail hereinafter with reference to the drawings. The embodiment described below has been set forth merely for preferred examples in nature, and are not intended to limit applications and use of the invention.

FIG. 1 is a view illustrating a case where hemodialysis treatment is performed by using a hemodialysis apparatus 1 of the embodiment of the present invention. The hemodialysis apparatus 1 includes a hemodialysis treatment administering unit 2; a controller 4 with an operation display unit 3; and an auxiliary memory (storage unit) 5 illustrated in FIG. 2. In the hemodialysis treatment administering unit 2 of such units, the hemodialysis treatment is actually provided. The controller 4 mainly includes an information processing unit of a computer, and controls the hemodialysis treatment administering unit 2. The auxiliary memory 5 is for storing and holding various data. In addition, the operation display unit 3 is for inputting data by a medical staff, and for displaying various information and the inputted data.

The hemodialysis treatment administering unit 2 includes a dialyzer (blood treating unit) 10 which is central to the hemodialysis treatment; an arterial blood circuit section 11 and a venous blood circuit section 12 which are connected to the dialyzer 10; a blood pump 13; a blood measuring instrument 14; and a dialysate circuit 15 for supplying dialysate to the dialyzer 10. The arterial blood circuit section 11, the venous blood circuit section 12, and the blood pump 13 form a blood circuit 16.

The dialyzer 10 includes a plurality of hollow fibers (not illustrated in the figure) accommodated in a cylindrical case. Patient's blood flows inside the hollow fibers, and dialysate flows through spaces between the hollow fibers within the case. Although not illustrated in the figure, blood inflow and outflow ports communicating with the insides of the hollow fibers, and dialysate inflow and outflow ports communicating with the spaces between the hollow fibers within the case are formed in the case of the dialyzer 10.

The arterial blood circuit section 11 includes an arterial-side puncture needle 11a, and is connected to the blood inflow port of the dialyzer 10. An arterial-side drip chamber 11b and the blood measuring instrument 14 are provided in the middle of the arterial blood circuit section 11. On the other hand, the venous blood circuit section 12 includes a venous-side puncture needle 12a, and is connected to the blood outflow port of the dialyzer 10. A venous-side drip chamber 12b is provided in the middle of the venous blood circuit section 12.

The blood measuring instrument 14 includes a hematocrit sensor for measuring a hematocrit value Ht indicating the proportion of blood volume which is occupied by red blood cells to the total blood volume. Although not illustrated in the figure, the hematocrit sensor includes, e.g., a light-emitting device implemented by LEDs etc.; and a light-receiving device implemented by photo diodes etc. Blood is irradiated with light from the light-emitting device to receive the light transmitting through the blood by the light-receiving device, thereby obtaining the hematocrit value Ht of the patient's blood flowing in the arterial blood circuit section 11. The hematocrit sensor continuously detects and outputs the hematocrit values Ht.

The blood pump 13 includes an electric motor (not illustrated in the drawing), and is a so-called "squeezing-type" pump in which a flexible tube forming the arterial blood circuit section 11 is squeezed to send blood from the arterial blood circuit section 11 to the venous blood circuit section 12. The rotational speed of the motor of the blood pump 13 is changed to adjust the flow rate of blood flowing in the blood circuit 16. The blood pump 13 is not limited to the squeezing-type pump, and other types of pumps may be used.

The dialysate circuit 15 includes a supply circuit section 17 connected to a dialysate supply device (not illustrated in the figure); a discharge circuit section 18; a body fluid removing pump 19; and a supply valve 20. The supply circuit section 17 is connected to the dialysate inflow port of the dialyzer 10. The discharge circuit section 18 is connected to the dialysate outflow port of the dialyzer 10. The supply valve 20 is provided in the supply circuit section 17, and is for adjusting the flow rate of dialysate flowing in the supply circuit section 17.

In addition, the body fluid removing pump 19 is provided in the discharge circuit section 18, an is for removing body fluid from blood. The body fluid removing pump 19 includes an electric motor (not illustrated in the figure). The body fluid removing pump 19 is driven to allow the volume of discharged body fluid to be larger than the volume of dialysate flowing into the dialyzer 10, thereby removing body fluid from blood flowing in the hollow fibers. The volume of body fluid removed by the body fluid removing pump 19 can be adjusted buy changing the rotational speed of the motor of the body fluid removing pump 19.

The controller 4 includes a central processing unit (CPU) (not illustrated in the figure); a main memory such as RAMs (not illustrated in the figure); a ROM (not illustrated in the figure) in which control programs are stored; and operation switches 28 and a display panel 29 provided in the operation display unit 3. The auxiliary memory 5 is connected to the controller 4 through a bus 6, and data communication is provided between the controller 4 and the auxiliary memory 5. The blood measuring instrument 14 is connected to the controller 4, and the hematocrit values obtained in the blood measuring instrument 14 are inputted to the controller 4. In addition, the controller 4 is connected to the blood pump 13, the body fluid removing pump 19, and the supply valve 20. The rotational speeds of the motors of the blood pump 13 and the body fluid removing pump 19 are changed based on output signals from the controller 4, thereby opening/closing the supply valve 20.

The operation switches 28 and the display panel 29 are provided on a front surface of the hemodialysis apparatus 1. The operation switches 28 are for inputting various data on a patient, performance data of the dialyzer 10, a hemodialysis time, the volume of body fluid to be removed, etc. for each patient. In addition, the display panel 29 is an indicator including a touch panel display. Operation buttons (described later) displayed on the display panel 29 are touched to input various data etc. The inputted data, the data stored in the auxiliary memory 5, the results calculated in the CPU, etc. are displayed on the display panel 29.

Although not illustrated in the figure, the hemodialysis apparatus 1 includes, e.g., a sensor for detecting pulse and blood pressure of a patient; a sensor for detecting a dialysate concentration; a sensor for detecting a dialysate temperature; a sensor for detecting venous pressure of the patient; and a sensor for detecting air bubbles entering the blood circuit 16. Such sensors are connected to the controller 4, and data detected in each of the sensors is inputted to the controller 4. In addition, the data inputted from the sensors is displayed on the display panel 29, thereby allowing medical staff to view such data.

The controller 4 performs predetermined processing according to the control programs based on the data obtained from various sensors, and the data inputted from the operation switches 28 and the operation buttons on the display panel 29, followed by controlling the motors of the blood pump 13 and the body fluid removing pump 19, and the supply valve 20.

A BV navigation function for adjusting the rate of change in circulating blood volume so as to approach a predetermined value is installed in the controller 4. Although described in detail later, as illustrated in FIG. 2, the BV navigation function includes a maximum change rate setting unit 31 for setting the maximum rate of change in circulating blood volume (maximum change rate $V_{MAX}$); an average change rate setting unit 32 for setting an average change rate (average change rate $V_{AVE}$) within the hemodialysis time for the full volume of circulating blood; and an adjustment line forming unit 33 for forming an adjustment line Q to be a reference line for adjusting the rate of change in circulating blood volume.

In the controller 4, an absolute value of the circulating blood volume itself is not used to control the hemodialysis apparatus 1, but a parameter which can obtain a relative change in circulating blood volume is used for such control. The parameter includes values changing as the hemodialysis treatment proceeds, such as a body fluid volume and a blood cell concentration in blood. When using the blood cell concentration for the control of the hemodialysis apparatus 1, it is preferable to use, e.g., the hematocrit value Ht; a blood volume (BV) value calculated from the hematocrit value Ht; and a %BV value which is obtained by dividing the BV value by a BV value when the hemodialysis treatment is started, and which is expressed in percentage. The parameter is not limited to the above, but various values may be used.

In the present embodiment, the hematocrit value Ht is used as the parameter relating to the circulating blood volume in the controller 4. In such a case, a circulating blood volume change ratio ΔBV(%) can be obtained by the following expression:

$$\text{Circulating Blood Volume Change Ratio } \Delta BV(\%) = (\text{Pretreatment Hematocrit Value } Ht_0 - \text{Measured Hematocrit Value } Ht_1) / \text{Measured Hematocrit Value } Ht_1 \times 100$$

Since the hematocrit sensor continuously detects and outputs the hematocrit values Ht, the circulating blood volume change ratio ΔBV(%) can be obtained in approximately real time.

The controller 4 displays a stop mode window; a hemodialysis mode window; a BV navigation operation window for operating the BV navigation function; etc. Specifically, when the hemodialysis apparatus 1 is stopped before starting the hemodialysis treatment, the stop mode window is displayed on the display panel 29. As illustrated in FIG. 3, the stop mode window includes a plurality of operation buttons 25 for various settings; and display areas 26 where a dialysate temperature etc. are displayed. The operation buttons 25 include a BV navigation button operated when using the BV navigation function. When operating the BV navigation button in the stop mode window, the controller 4 displays the BV navigation operation window (illustrated in FIG. 4) on the display panel 29. Although not illustrated in the figure, when starting the hemodialysis treatment by the hemodialysis apparatus 1, the controller 4 displays the hemodialysis mode window on the display panel 29. The hemodialysis mode window includes various operation buttons; and display areas where a time elapsed since the hemodialysis treatment is started, and the dialysate temperature are displayed.

The controller 4 sets the maximum change rate $V_{MAX}$ of the circulating blood volume in the maximum change rate setting unit 31, and sets the average change rate $V_{AVE}$ in the average change rate setting unit 32. Subsequently, the controller 4 forms the adjustment line in the adjustment line forming unit 33. A shape of the formed adjustment line can be changed before and after starting the hemodialysis treatment.

First, a case where the maximum change rate $V_{MAX}$ of the circulating blood volume is set in the maximum change rate setting unit 31 will be described. A plurality of options of data associated with the maximum change rate $V_{MAX}$ are stored in the auxiliary memory 5. As in the BV navigation operation window (BV navigation selection 1) illustrated in FIG. 4, the data associated with the maximum change rate $V_{MAX}$ has 12 possible options which are "-5% for 60 min," "-5% for 90 min," "-5% for 120 min," "-10% for 60 min," "-10% for 90 min," "-10% for 120 min," "-15% for 60 min," "-15% for 90 min," "-15% for 120 min," "-20% for 60 min," "-20% for 90 min," and "-20% for 120 min."

For example, the option "-5% for 60 min" means that the circulating blood volume when starting the hemodialysis treatment is decreased by 5% for 60 minutes. That is, the parameter "-5%" is the circulating blood volume change ratio ΔBV(%). The parameter "60 min" is a change time t (minutes), and the change time t is set so as to be shorter than a general hemodialysis time T (approximately 4-5 hours). The change ratio ΔBV(%) and the change time t are used to obtain the volume of change. The volume of change is a difference between a patient's circulating blood volume when starting the hemodialysis treatment and a target circulating blood volume when terminating the hemodialysis treatment, and a greater absolute value of the change ratio ΔBV(%) and a longer change time t result, in a greater volume of change. In addition, the target circulating blood volume is different depending on patient's conditions etc., and is set for each patient. The data associated with the maximum change rate $V_{MAX}$ can be selected based on the target circulating blood volume.

Each of the maximum change rates $V_{MAX}$ is a value to which a sufficient safety margin is provided so that the change rate does not become too fast for a typical patient.

The data associated with the maximum change rate $V_{MAX}$ is not limited to the above. The change ratio ΔBV (%) may be arbitrarily changed within, e.g., a range from -5% to -20%. In addition, the change time t may be arbitrarily changed within, e.g., a range from 60 minutes to 120 minutes.

The maximum change rate setting unit 31 displays selection buttons 34 corresponding to the 12 possible options of the data associated with the maximum change rate $V_{MAX}$ on the display panel 29. Subsequently, the maximum change rate setting unit 31 reads the data corresponding to the operated selection button 34 from the auxiliary memory 5, and sets such data as the maximum change rate $V_{MAX}$. As illustrated in FIG. 5, the maximum change rate setting unit 31 plots a graph based on the set data associated with the maximum change rate $V_{MAX}$. A horizontal axis of the graph represents a time (minutes), and a vertical axis represents the circulating blood volume change ratio ΔBV(%). A slope of the graph represents the maximum change rate $V_{MAX}$, and therefore the graph defines a maximum change rate representing line S which represents the maximum change rate $V_{MAX}$. The maximum change rate representing line S is a straight line.

Next, a case where the average change rate $V_{AVE}$ of the circulating blood volume is set in the average change rate setting unit 32 will be described. The average change rate $W_{AVE}$ is calculated by using the hemodialysis time T inputted by a medical staff, and the data associated with the maximum change rate $V_{MAX}$ set in the maximum change rate setting unit 31; and is a change rate averaged within the hemodialysis time T when the circulating blood volume is changed by the volume of change (the volume reduced to allow the circulating blood volume when starting the hemodialysis treatment to reach the target circulating blood volume) within the hemodialysis time T. As illustrated in FIG. 5, the hemodialysis time T is longer than the change time t, a slope of an average change rate representing line R which represents the average change rate $V_{AVE}$ is more gradual than that of the maximum change rate representing line S. The average change rate representing line R is a straight line.

Next, a case where the adjustment line Q (illustrated in FIG. 5) is formed in the adjustment line forming unit 33 will be described. The adjustment line Q is for adjusting the change rate so that the rate of change in circulating blood volume approaches a change rate equal to or slower than the maximum change rate $V_{MAX}$. The adjustment line Q is formed within a formation area W between the maximum change rate representing line S and the average change rate representing line R. The formation area W contains areas on the maximum change rate representing line S and the average change rate representing line R.

Specifically, the adjustment line Q is formed by using a plurality of adjustment line forming blocks A (illustrated in FIG. 6) arranged in a direction representing a lapse of the hemodialysis time T. For the data associated with the maximum change rate $V_{MAX}$, 20 adjustment line forming blocks A are set for the options "-5% for 60 min," "-10% for 60 min," "-15% for 60 min," and "-20% for 60 min;" 30 adjustment line forming blocks A are set for the options for 90 min," "-10% for 90 min," "-15% for 90 mm," and "-20% for 90 min;" and 40 adjustment line forming blocks A are set for the options "-5% for 120 min," "-10% for 120 min," "-15% for 120 min," and "-20% for 120 min." Such numbers of adjustment line forming blocks A are stored in association with the data associated with the maximum change rate $V_{MAX}$. The number of adjustment line forming blocks A may be changed by a user.

Each of the adjustment line forming blocks A has a quadrilateral shape with a diagonal line defined by a line segment S' which is one of line segments formed by equally dividing the maximum change rate representing line S, thereby realizing the adjustment line forming blocks A having the same size. As illustrated in FIG. 7, the horizontal length of the adjustment line forming block A represents a time (unit time) obtained by dividing the change time t contained in the data associated with the maximum change rate. $V_{MAX}$ by the number of adjustment line forming blocks A; and the vertical length of the adjustment line forming block A represents a change ratio (unit change ratio) obtained by dividing the change ratio ΔBV(%) contained in the data associated with the maximum change rate $V_{MAX}$ by the number of adjustment line forming blocks A. For example, if the maximum change rate $V_{MAX}$ is set to the option "-5% for 60 min," the number of adjustment line forming blocks A is 20. Thus, the unit time is represented by an expression 60 min/ 20 = 3 min, and the unit change ratio is represented by an expression -5% / 20 = -0.25%.

If a part of the adjustment line Q corresponding to a first half of the hemodialysis treatment is formed so as to be coincident, with the maximum change rate representing line S, the adjustment line forming blocks A are arranged with no clearance therebetween on the maximum change rate representing line S to connect the diagonal lines of the adjustment line forming blocks A to each other as illustrated in FIG. 6, thereby defining the first part of the adjustment line Q extending so as to connect the adjacent adjustment line forming blocks A.

On the other hand, if the adjustment line Q which is not coincident with the maximum change rate representing line S is formed, time adjustment blocks B are combined with the adjustment line forming blocks A as illustrated in FIG. 8. In order to combine the time adjustment blocks B with the adjustment line forming blocks A, a first clearance C1 is formed on a front side (front side in a direction opposite to the direction representing the lapse of the hemodialysis time T) of a first adjustment line forming block A1, followed by forming second to twentieth clearances C2-C20 between the first adjustment lin forming block A1 and a second adjustment line forming block A2, between the second adjustment line forming block A2 and a third adjustment line forming block A3, ..., and between the nineteenth adjustment line forming block A19 and a twentieth adjustment line forming block A20, respectively. Subsequently, the time adjustment block(s) B are formed in each of the first to twentieth clearances C1-C20.

The time adjustment block B is for making up a difference between the change time t which is set when setting the maximum change rate $V_{MAX}$ and the hæmodialysis time T. As illustrated in FIG. 5, the difference between the change time t and the hemodialysis time T is an adjustment time, As illustrated in FIG. 9, the horizontal length of the time adjustment block B represents a time (unit adjustment time) obtained by dividing the adjustment time by the number of time adjustment blocks B.

As illustrated in FIG. 10, there are A to F patterns relating to the number of time adjustment blocks B to be arranged, and such patterns are stored in the auxiliary memory 5. The adjustment line forming unit 33 displays selection buttons 35 corresponding to the data associated with the A to F patterns as the BV navigation operation window on the display panel 29. Subsequently, the adjustment line forming unit 33 reads the pattern data corresponding to the operated selection button 35 from the auxiliary memory 5, and arranges the time adjustment blocks B based on such pattern data.

A method for forming the adjustment lines Q in the A to F patterns will be described below. Note that the maximum change rate $V_{MAX}$ is set to the option "-10% for 60 min."

As illustrated in FIG. 11, in the A pattern, 20 adjustment line forming blocks A1-A20 are arranged at equal intervals, and, as illustrated in FIG. 12, a single time adjustment block B is arranged in each of first to twentieth clearances C1-C20. Thus, in the A pattern, the number of time adjustment blocks B is 20. In the A pattern, if the hemodialysis time T is 240 minutes, the adjustment time is represented by an expression 240 min - 60 min = 180 min as illustrated in FIG. 11. Then, when the adjustment time of 180 min is divided by the number of time adjustment blocks B which is 20, the unit adjustment time is 9 minutes as illustrated in FIG. 12, and serves as the horizontal length of the time adjustment block B. The time adjustment block B arranged in the twentieth clearance C20 has the horizontal length which allows absorption of temporal errors caused when arranging the 20 time adjustment blocks and the 20 adjustment line forming blocks A1-A20, and such horizontal length differs from those of the time adjustment blocks B arranged in other clearances. If no temporal error is caused, the horizontal length of the time adjustment block B arranged in the twentieth clearance C20 is equal to those of the time adjustment blocks B arranged in other clearances.

When forming the adjustment line Q in the A pattern, a diagonal line (indicated by a dashed line in FIG. 12) of a block formed by combining the first time adjustment block B and the first adjustment line forming block A1, a diagonal line of a block formed by combining the second time adjustment block B and the second adjustment line forming block A2, ..., and a diagonal line of a block formed by combining the twentieth time adjustment block B and the twentieth adjustment line forming block A20 are connected to each other, thereby forming the adjustment line Q extending so as to connect, the adjacent adjustment line forming blocks A. In the A pattern, the adjustment line Q is formed on the average change rate representing line R. This allows a slope of the adjustment line Q to be more gradual than that of the maximum change rate representing line S.

The 13 pattern will be described with reference to FIGS. 8 and 13. As illustrated in Fig. 8, in the B pattern, a single time adjustment block B is arranged in a first clearance C1; two time adjustment blocks B are arranged in a second clearance C2; and three time adjustment blocks B are arranged in a third clearance C3. In this manner, 1-20 time adjustment block(s) B are arranged in each of the first to twentieth clearances C1-C20. That is, in the B pattern, the number of time adjustment blocks B to be arranged in the first to twentieth clearances C1-C20 increases in arithmetic progression, and the total number of time adjustment blocks B is represented by an expression 1 + 2 + 3 + 4 + ... + 19 + 20 = 210. In the B pattern, if the hemodialysis time T is 240 minutes, 0.857 minutes obtained by dividing the adjustment time of 180 minutes by the number of time adjustment blocks B which is 210 is the unit adjustment time.

When forming the adjustment line Q in the B pattern, a diagonal line (indicated by a dashed line in FIG. 8) of a block formed by combining the time adjustment block B arranged in the first clearance C1 and a first adjustment line forming block A1, a diagonal line of a block formed by combining all of the time adjustment blocks B arranged in the second clearance C2 and a second adjustment line forming block A2, ..., and a diagonal line of a block formed by combining all of the time adjustment blocks B arranged in the twentieth clearance C20 and a twentieth adjustment line forming block A20 are connected to each other, thereby forming the adjustment line Q.

The C pattern will be described with reference to FIG. 14. In the C pattern, the number of time adjustment blocks (not illustrated in the figure) to be arranged in first to twentieth clearances C1-C20 increases in difference sequence (1, 2, 4, 7, ... ), and the total number of time adjustment blocks is 1,306. In the C pattern, if the hemodialysis time T is 240 minutes, 0.138 minutes obtained by dividing the adjustment time of 180 minutes by the number of time adjustment, blocks which is 1,306 is the unit adjustment time. The adjustment line Q is formed as in the B pattern.

The D pattern will be described with reference to FIG. 15. In the D pattern, the number of time adjustment blocks (not illustrated in the figure) to be arranged in first to twentieth clearances C1-C20 increases in Fibonacci sequence (1, 2, 3, 5, 8, 13, ...), and the total number of time adjustment blocks is 28,655, In the D pattern, if the hemodialysis time T is 240 minutes, 0,0062186 minutes obtained by dividing the adjustment time of 180 minutes by the number of time adjustment blocks which is 28,655 is the unit adjustment time. The adjustment line Q is formed as in the B pattern.

The E pattern will be described with reference to FIG. 16. In the E pattern, the number of time adjustment blocks (not illustrated in the figure) to be arranged in first to twentieth clearances C1-C20 increases in factorial sequence (1, $2 \times 2$, $2 \times 2 \times 2$, $2 \times 2 \times 2 \times 2$, ...), and the total number of time adjustment blocks is 1,048,575. In the E pattern, if the hemodialysis time T is 240 minutes, 0.00017166 minutes obtained by dividing the adjustment time of 180 minutes by the number of time adjustment blocks which is 1,048,575 is the unit adjustment time. The adjustment line Q is formed as in the B pattern. The B to E patterns are sequence patterns.

The F pattern will be described with reference to FIG. 17. In the F pattern, the number of adjustment line forming blocks A is reduced by half, and such blocks are arranged at equal intervals within the hemodialysis time T. In the F pattern, the number of time adjustment blocks (not illustrated in the figure) is 10 which is half of that in the A pattern. Diagonal lines of the time adjustment blocks and of the adjustment line forming blocks A are connected to form the adjustment line Q.

The present embodiment has the six patterns relating to the number of time adjustment blocks B to be arranged, which are the A to F patterns, but is not limited to the above. For example, another pattern made by combining the A to F patterns may be stored in advance, or a medical staff may make a pattern other than the above-described patterns to store such a pattern in the auxiliary memory 5. In addition, a part of the pattern which has been selected once may be changed to store it as another pattern in the auxiliary memory 5. Further, the time adjustment block B may not be arranged in a part of the first to twentieth clearances C1-C20.

As illustrated in FIG. 18, the controller 4 displays the maximum change rate representing line S, the average change rate representing line R, and the adjustment line Q which have been obtained as described above, as the BV navigation operation window on the display panel 29. At such a state, although not illustrated in FIG. 18, the adjustment line forming blocks A are also displayed. Shift buttons 40-43 and various operation buttons 44, 45, and 47-50 are displayed on the BV navigation operation window. Alter the formation of the adjustment line Q, such buttons 44, 45, and 47-50 are operated to change a shape of the adjustment line Q. The control for changing the shape of the adjustment line Q is performed in the adjustment line forming unit 33 of the controller 4.

First, a case where the shape of the adjustment line Q is changed before starting the hemodialysis treatment will be described with reference to FIGS. 19 and 20. When operating the shift button 40 indicated by an up-pointing arrow on the BV navigation operation window on which the maximum change rate representing line S, the average change rate representing line R, and the adjustment line Q are displayed, a change ratio representing line L1 which represents the change ratio ΔBV(%) set in the maximum change rate setting unit 31 is upwardly shifted as illustrated in FIG. 19(a), thereby decreasing the absolute value of the change ratio ΔBV(%). Then, the vertical length of the adjustment line forming blocks A is set so as to be shorter (the absolute value of the unit change ratio is corrected in the decrease direction), thereby changing the shape of the adjustment line forming blocks A. The adjustment line Q is regenerated based on such adjustment line forming blocks A.

As illustrated in FIG. 19(b), when operating the shift button 41 indicated by a down-pointing arrow, the change ratio representing line L1 is downwardly shifted, thereby increasing the absolute value of the change ratio ΔBV(%). Then, the vertical length of the adjustment line forming blocks A is set so as to be longer (the absolute value of the unit change ratio is corrected in the increase direction), the adjustment line Q is regenerated based on such adjustment line forming blocks A. In addition, when the change ratio representing line L1 is shifted as described above, the change ratio ΔBV (%) is changed, thereby changing the average change rate $V_{AVE}$ in response thereto.

In addition, as illustrated in FIG. 20(a), when operating the shift button 42 indicated by a left-pointing arrow, a termination representing line L2 which represents a termination of the hemodialysis treatment is shifted to a left side (a side to which the hemodialysis time T is shortened), thereby shortening the hemodialysis time T. Then, the adjustment time is decreased, and the horizontal length of the time adjustment blocks B is set to be shorter (the unit adjustment time is corrected in the decrease direction), thereby changing the shape of the time adjustment blocks B. Consequently, the adjustment, line Q is regenerated.

As illustrated in FIG. 20(b), when operating the shift button 43 indicated by a right-pointing arrow, the termination representing line L2 is shifted to a right side (a side to which the hemodialysis time T is extended), thereby extending the hemodialysis time T. Then, the adjustment time is increased, and the horizontal length of the time adjustment blocks B is set to be longer (the unit adjustment time is corrected in the increase direction), thereby regenerating the adjustment line Q. When shifting the termination representing line L2, the shape of the adjustment line forming blocks A is not changed.

When operating the BV start point changing button 48, a BV navigation start point representing line L3 which represents a start point of the BV navigation function is displayed as illustrated in FIG. 21. When operating the left and right shift buttons 42 and 43 in such a state, the BV navigation start, point representing line L3 is shifted in the horizontal direction, thereby changing the start, point of the BV navigation, function.

When operating the BV first-half period changing button 49, a boundary L4 dividing a period of time for which the BV navigation is performed, into first and second halves is displayed as illustrated in FIG. 22. When operating the left and right shrift buttons 42 and 43 in such a state, the boundary L4 is shifted in the horizontal direction, thereby changing the first-half period. During the second-half period of the hemodialysis treatment, intermittent infusion and Na infusion are performed. The start timings and volumes of such infusions are set separately.

When operating the BV second-half period changing button 50, a second-half period end point representing line L5 which represents an end point of the second-half period is displayed as illustrated in FIG. 23. When operating the left and right shift buttons 42 and 43 in such a state, the second-half period end point representing line L5 is shifted in the horizontal direction, thereby changing the second-half period.

The blocks A1-A19 other than the twentieth adjustment, line forming block A20 can be shifted to change the shape of the adjustment line Q. In such a case, by operating the left and right shift buttons 45, a cursor Z for selecting the adjustment line forming block A1-A19 is displayed to shift in the horizontal direction as illustrated in FIG. 24. When pressing an enter button 47 after the cursor Z is shifted to a desired one of the adjustment line forming blocks A1-A19, such an adjustment line forming block A enters a selected state. In such a state, when operating the shift buttons 40-43, the adjustment line forming block A is shifted in the direction(s) corresponding to the operated shift button(s) 40-43. When upwardly shifting the adjustment line forming block A, a potion of the adjustment line Q subsequent to such a block is upwardly parallel-shifted in response thereto without changing the shape of the adjustment line Q. When shifting the adjustment line forming block A downwardly, to the left side, or to the right side, the subsequent, portion of the adjustment line Q is also shifted in the same manner. As described above, the adjustment line forming block A1-A19 other than the twentieth bock A20 is shifted, thereby regenerating the adjustment line Q.

When operating the final block changing button 44 in the BV navigation operation window, a cursor is displayed so as to overlap with the final adjustment line forming block (twentieth adjustment line forming bock A20) corresponding to the end point of the hemodialysis time T. When operating the shift button(s) 40-43 in such a state, the adjustment line forming block A20 is shifted in the direction(s) corresponding to the operated shift button(s) 40-43. When upwardly shifting the twentieth adjustment line forming block A20, the change ratio representing line L1 (illustrated in FIG. 19) is upwardly shifted in response thereto, thereby decreasing the absolute value of the change ratio ΔBV(%). When downwardly shifting the twentieth adjustment line forming block A20, the change ratio representing line L1 is downwardly shifted in response thereto, thereby increasing the absolute value of the change ratio ΔBV(%). Consequently, the adjustment line Q is reformed.

When shifting the twentieth adjustment line forming block A20 to the left side, the termination representing line L2 (illustrated in FIG. 20) is shifted to the left side in response thereto, thereby shortening the hemodialysis time T. When shifting the twentieth adjustment line forming block A20 to the right side, the termination representing line L2 is shifted to the right side in response thereto, thereby extending the hemodialysis time T. Consequently, the adjustment line Q is regenerated.

Next, a case where the shape of the adjustment line Q is changed after the hemodialysis treatment is started will be described. As in the case where the shape is changed before starting the hemodialysis treatment, if the shape is changed after the hemodialysis treatment is started, the shape of the adjustment line Q can be changed by shifting the adjustment line forming block A, and the shape of the adjustment line Q in an area where the dialysis time T has elapsed is not changed.

That is, as illustrated in FIG. 25, if the hemodialysis time T of, e.g., 65 minutes has elapsed since the hemodialysis treatment is started, and the hemodialysis time T has reached a point before a twelfth adjustment line forming block A12, the twelfth adjustment line forming block A12 is locked in order not to be shifted and be changed in its shape, the adjustment line forming blocks A13-A20 subsequent to the adjustment line forming block A12 are shifted to regenerated the adjustment line Q. As illustrated in the figure, when the twentieth adjustment line forming block A20 is shifted to the right side (direction indicated by an arrow in the figure) to extend the hen-zodialysis time T, the time adjustment blocks B subsequent to the thirteenth clearance C13 is set so that the horizontal length is increased by the length equivalent to the extended time, thereby changing the shape of the adjustment line Q in response thereto. When the twentieth adjustment line forming block A20 is shifted to the left side (direction in which the hemodialysis time T is shortened), the horizontal length of the time adjustment blocks B subsequent to the thirteenth clearance C13 is set so as to be shorter, thereby changing the shape of the adjustment line Q.

As illustrated in FIG. 26, when downwardly shifting the twentieth adjustment line forming block A20 to change the change ratio ΔBV(%), the vertical length of the adjustment line forming blocks A13-A20 subsequent to the twelfth adjustment, line forming block A12 is set so as to be longer, thereby changing the shape of the adjustment line Q in response thereto. When upwardly shifting the twentieth adjustment line forming block A20, the vertical length of the adjustment line forming blocks A13-A20 is set so as to be shorter, thereby changing the shape of the adjustment line Q in response thereto.

When shifting the adjustment line forming block A1-A19 other than the twentieth adjustment line forming block A20, the adjustment line forming block A1-A19 is selected by the cursor to operate the shift buttons 45 as in the shape change before starting the hemodialysis treatment. At such a state, as illustrated in FIG. 27, e.g., when the eleventh adjustment, line forming block A11 is locked, only the adjustment line forming block A12-A20 subsequent to the eleventh adjustment line forming block A11 can be selected. When shifting the twelfth adjustment line forming block A12 to the right side, the twelfth to twentieth adjustment line forming blocks A12-A20 are parallel-shifted to the right side, thereby extending the hemodialysis time T. Thus, the shape of a portion of the adjustment line Q subsequent to the adjustment line forming block A12 is not changed. When shifting the twelfth adjustment line forming block A12 to the left side, the twelfth to twentieth adjustment line forming blocks A12-A20 are parallel-shifted to the left side, thereby shortening the hemodialysis time T. At such a state, the twelfth adjustment line forming block A12 is not shifted to the left side with respect to the eleventh adjustment line forming block A11.

When downwardly shifting the twelfth adjustment line forming block A12, the twelfth to twentieth adjustment line forming blocks A12-A20 are downwardly parallel-shifted. When upwardly shifting the twelfth adjustment line forming block A12, the twelfth to twentieth adjustment line forming blocks A12-A20 are upwardly parallel-shifted. At such a point, the twelfth adjustment line forming block A12 is not shifted to above the eleventh adjustment line forming block A11.

In order to stop the BV navigation function, a stop button (not illustrated in the figure) displayed in the BV navigation operation window is operated to stop such a function. In order to restart the BV navigation function after the temporary stop, a restart button (not illustrated in the figure) is operated to restart such a function.

Next, a case where the hemodialysis apparatus 1 configured as described above is used will be described. First, a medical staff prepares for the hemodialysis treatment by connecting the blood circuit 16 to a patient and the dialyzer 10, and connecting the dialysate circuit 15 to the dialyzer 10. At such a state, as illustrated in FIG. 3, the stop mode window is displayed on the display panel 29 of the hemodialysis apparatus 1, and the medical staff operates the operation buttons to input the hemodialysis time, the volume of body fluid to be removed, etc. Subsequently, when the medical staff operates the BV navigation button, the BV navigation operation window is displayed on the display panel 29 as illustrated in FIG. 4. A button corresponding to the maximum change rate $V_{MAX}$ suitable to the patient is selected from the selection buttons 34 of such an operation window, and then is operated. The maximum change rate $V_{MAX}$ is obtained depending on patient's conditions etc. in advance, and can be set regardless of medical staff's experiences. In addition, the number of adjustment line forming blocks A is selected at such a state.

When operating the operation button of the maximum change rate $V_{MAX}$, the BV navigation operation window illustrated in FIG. 10 is displayed on the display panel 29. The selection buttons 35 corresponding to the patterns (A to F patterns) relating to the number of time adjustment blocks B to be arranged are displayed on such a window. From such selection buttons 35, the medical staff operates the selection button 35 corresponding to the pattern which seems to be suitable to the patient.

Then, the maximum change rate representing line S is formed in the maximum change rate setting unit 31 while forming the average change rate representing line R in the average change rate setting unit 32. Further, in the adjustment line forming unit 33, the adjustment line Q corresponding to the selected pattern is formed. As illustrated in FIG. 18, the maximum change rate representing line S, the average change rate representing line R, and the adjustment line Q are displayed on the display panel 29. At such a state, the adjustment line forming blocks A are also displayed. This allows the medical staff to visually view the formed adjustment line Q.

If the formed adjustment line Q is allowed, the hemodialysis treatment is started. If the medical staff wishes to change the shape of the adjustment line Q, the shift buttons 40-43 etc. are operated to change such a shape. When starting the hemodialysis treatment, the hemodialysis treatment administering unit 2 is controlled so that the rate of change in circulating blood volume approaches the change rate which is set as the adjustment line Q. A slope in each section of the adjustment line Q is equal to or less than a slope of the maximum change rate representing line S, and therefore the rate of change in circulating blood volume is not increased to such an extent as to cause a discomfort of the patient.

When the medical staff wishes to change the shape of the adjustment line Q, a particular adjustment line forming block A may be simply shifted. This allows other adjustment line forming blocks A and time adjustment blocks B to be automatically shifted, and allows the change in the shape of the adjustment line Q in order to form the adjustment line Q. Thus, an operation by the medical staff is simple.

As described above, according to the hemodialysis apparatus 1 of the present embodiment, the maximum change rate $V_{MAX}$ of the patient's circulating blood volume is set, and the adjustment line Q for adjusting the circulating blood volume change rate is formed so as to be equal to or less than the maximum change rate $V_{MAX}$. Thus, the circulating blood volume change rate is not increased to such an extent as to cause the patient's discomfort, and the suitable adjustment line Q can be easily formed even by an inexperienced medical staff. This reduces a risk to a patient during the hemodialysis treatment, and allows an efficient hemodialysis treatment.

The maximum change rate representing line S which represents the maximum change rate $V_{MAX}$ of the circulating blood volume, and the adjustment line Q are displayed on the display panel 29. Thus, a medical staff can visually view whether or not the adjustment line Q is correctly formed, thereby further improving safety of a patient.

The plurality of options of the maximum change rate $V_{MAX}$ are stored in the auxiliary memory 5, and any option is simply selected from the plurality of options of the maximum change rate $V_{MAX}$ in order to set the maximum change rate $V_{MAX}$. Thus, the maximum change rate $V_{MAX}$ suitable to patient's conditions can be easily set.

A plurality of adjustment line forming blocks A arranged in the direction representing the lapse of the hemodialysis time T are arranged so as to be apart from each other, and the adjustment line Q extending so as to connect the adjacent adjustment line forming blocks A is formed. Thus, the rate of change in circulating blood volume can be arbitrarily changed depending on the clearance size between the adjustment line forming blocks A.

The average change rate setting unit 32 sets the average change rate $V_{AVE}$ of the circulating blood volume within the hemodialysis time so that the circulating blood volume when starting the hemodialysis treatment reaches the target circulating blood volume at a preset termination point of the hemodialysis treatment. This allows the adjustment line Q to be formed, considering the average change rate $V_{AVE}$ reaching the target circulating blood volume upon the termination of the hemodialysis treatment. Thus, an unnecessary extension of the hemodialysis time can be reduced.

The time adjustment block(s) B are arranged between the adjacent adjustment line forming blocks A. Thus, by simply changing the number of time adjustment blocks B to be arranged between the adjustment line forming blocks A, the size of the clearance C between the adjacent adjustment line forming blocks A can be changed, thereby easily changing the rate of change in circulating blood volume.

The pattern relating to the number of time adjustment blocks B to be arranged between the adjacent adjustment line forming blocks A is simply changed, thereby easily changing the rate of change in circulating blood volume so as to correspond to such a pattern.

Any pattern can be selected with a plurality of patterns relating to the number of time adjustment blocks B to be arranged being displayed on the display panel 29. Thus, ease of use when selecting the pattern relating to the number of time adjustment blocks B to be arranged can be improved.

Any adjustment line forming block A can be shifted after the formation of the adjustment line Q, thereby changing the shape of the adjustment line Q which has been formed once, so as to be suitable to patient's conditions. Thus, the adjustment line Q can be more suitably formed.

After the hemodialysis time T is changed, the clearances C between the adjustment line forming blocks A are adjusted so that the pre-change hemodialysis time T becomes coincident with the changed hemodialysis time T. Thus, when a medical staff changes the hemodialysis time T, the adjustment line Q corresponding to the changed hemodialysis time T can be formed without an operation for adjusting the clearances C between the adjustment line forming blocks A, thereby improving the ease of use.

During the hemodialysis treatment, only the adjustment line forming blocks A subsequent to a point at which the hemodialysis time T has elapsed can be shifted. Thus, the shape of the adjustment line Q up to a point at which the hemodialysis treatment has been performed can be remained, and the shape of the subsequent portion of the adjustment line Q can be set based on such a shape.

The adjustment line forming blocks A are displayed on the display panel 29, thereby improving the ease of use when shifting the adjustment line forming blocks A.

In the foregoing embodiment, the average change rate $V_{AVE}$ is obtained in the controller 4, and then the adjustment line Q is formed. However, the adjustment line Q may be formed without obtaining the average change rate $V_{AVE}$.

INDUSTRIAL APPLICABILITY

As described above, the hemodialysis apparatus of the present invention is suitable to easily adjust the rate of change in circulating blood volume so as to reach an appropriate value even by an inexperienced medical staffs.

## Claims

**1.** A hemodialysis apparatus performing a predetermined treatment by extracorporeally circulating patient's blood, comprising:

a hemodialysis treatment administering unit including a blood treating unit for treating patient's blood; and
a controller for controlling the hemodialysis treatment administering unit,
wherein the controller includes a maximum change rate setting unit for setting the maximum rate of change in circulating blood volume, and an adjustment line forming unit for forming an adjustment line for adjusting the rate of change in circulating blood volume so as to be equal to or less than the maximum change rate set in the maximum change rate setting unit; and controls the hemodialysis treatment administering unit based on the adjustment line formed in the adjustment line forming unit.

**2.** The hemodialysis apparatus of claim 1, wherein
the controller displays the adjustment line on an indicator of the controller.

**3.** The hemodialysis apparatus of claim 2, wherein
the controller displays a maximum change rate representing line which represents the maximum change rate, on the indicator.

**4.** The hemodialysis apparatus of any one of claims 1-3, wherein
the controller includes a storage unit for storing a plurality of options of the maximum change rate; and
the maximum change rate setting unit sets one of the plurality of options of the maximum change rate, which are stored in the storage unit.

**5.** The hemodialysis apparatus of any one of claims 1-4, wherein
the controller includes an average change rate setting unit for setting the average rate of change in circulating blood volume within a total hemodialysis time; and
the adjustment line forming unit forms the adjustment line between an average change rate representing line which represents the average change rate set in the average change rate setting unit, and the maximum change rate representing line.

**6.** The hemodialysis apparatus of claim 5, wherein
the average change rate setting unit sets the average rate of change in circulating blood volume within the hemodialysis time so that a circulating blood volume when starting hemodialysis treatment reaches a target circulating blood volume at a preset termination point of the hemodialysis treatment.

**7.** The hemodialysis apparatus of any one of claims 1-6, wherein
the adjustment line forming unit arranges a plurality of adjustment line forming blocks in a direction representing a lapse of the hemodialysis time; and arranges at least one of the adjustment line forming blocks so as to be apart from the adjacent adjustment line forming block in the direction representing the lapse of the hemodialysis time in order to form the adjustment line extending so as to connect the adjacent adjustment line forming blocks.

**8.** The hemodialysis apparatus of claim 7, wherein
a plurality of options of data associated with the number of adjustment line forming blocks are stored in the controller; and
any data option can be selected from the options of the data associated with the number of adjustment line forming blocks.

**9.** The hemodialysis apparatus of claim 7 or 8, wherein
time adjustment block(s) are arranged between the adjacent adjustment line forming blocks.

**10.** The hemodialysis apparatus of claim 9, wherein
the adjustment line forming unit can change a pattern relating to the number of time adjustment blocks to be arranged.

**11.** The hemodialysis apparatus of claim 10, wherein
the adjustment line forming unit displays a plurality of patterns relating to the number of time adjustment blocks to be arranged, on the indicator of the controller; and can select one of the displayed patterns.

**12.** The hemodialysis apparatus of claim 10 or 11, wherein
the pattern relating to the number of time adjustment blocks to be arranged is a pattern according to a predetermined sequence.

**13.** The hemodialysis apparatus of any one of claims 9-12, wherein
the time adjustment block adjacent to the adjustment line forming block positioned at the end in the direction representing the lapse of the hemodialysis time is formed so as to absorb temporal errors caused due to arrangement of other time adjustment blocks.

**14.** The hemodialysis apparatus of any one of claims 7-13, wherein
the adjustment line forming unit can change a shape of the adjustment line by shifting any adjustment line forming block after formation of the adjustment line.

**15.** The hemodialysis apparatus of claim 14, wherein
the adjustment line forming unit changes the hemodialysis time by shifting the final adjustment line forming block corresponding to an end point of the hemodialysis time; and adjusts clearances between the adjacent adjustment line forming blocks so that the pre-change hemodialysis time becomes coincident with the changed hemodialysis time.

**16.** The hemodialysis apparatus of claim 14 or 15, wherein
a shape of the adjustment line forming block is changed.

**17.** The hemodialysis apparatus of any one of claims 14-16, wherein
a shape of the time adjustment block is changed.

**18.** The hemodialysis apparatus of any one of claims 14-17, wherein
the adjustment line forming unit can shift only the adjustment line forming blocks subsequent to a point at which the hemodialysis time has elapsed, during the hemodialysis treatment.

**19.** The hemodialysis apparatus of any one of claims 14-18, wherein
the adjustment line forming unit can change the shape of the adjustment line by parallel-shifting a portion of the adjustment line subsequent to the point at which the hemodialysis time has elapsed with its shape being maintained.

**20.** The hemodialysis apparatus of any one of claims 7-19, wherein the adjustment line forming unit displays the adjustment line forming blocks on the indicator of the controller.

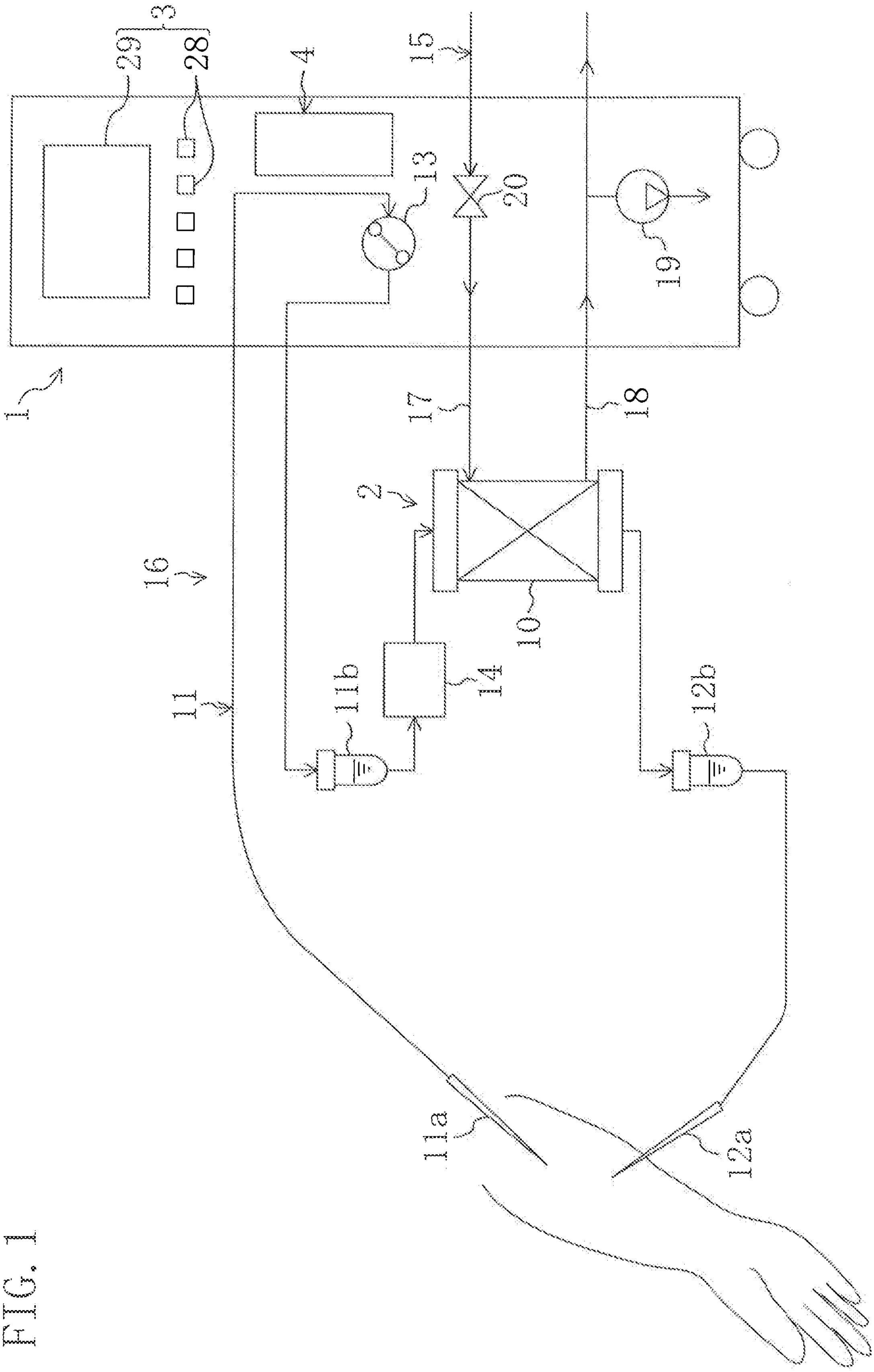


FIG. 1

FIG. 2

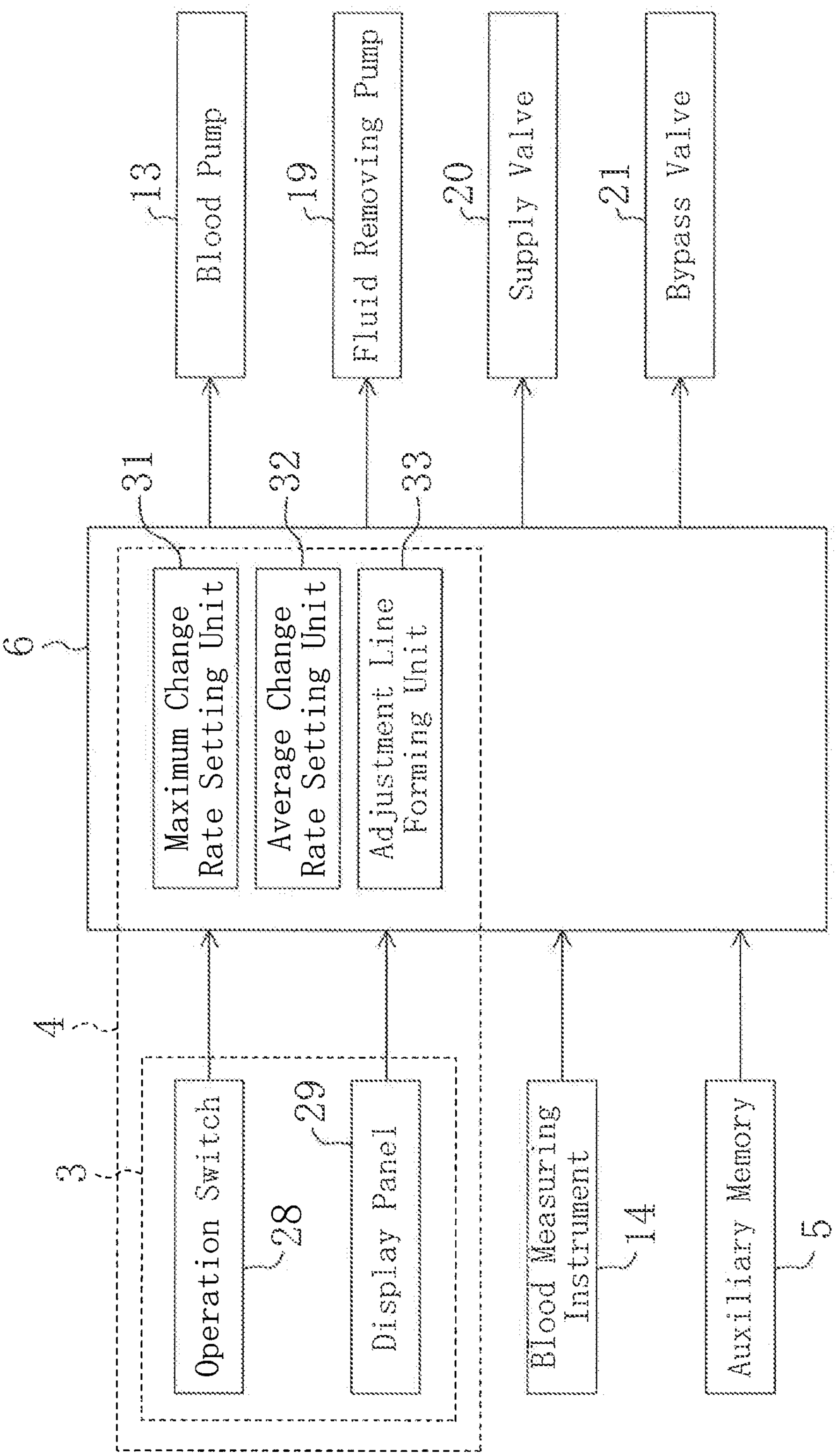
13
Blood Pump
19
Fluid Removing Pump
20
Supply Valve
21
Bypass Valve
31
32
33
6
Maximum Change Rate Setting Unit
Average Change Rate Setting Unit
Adjustment Line Forming Unit
4
3
Operation Switch
28
29
Display Panel
Blood Measuring Instrument
14
Auxiliary Memory
5

FIG. 3

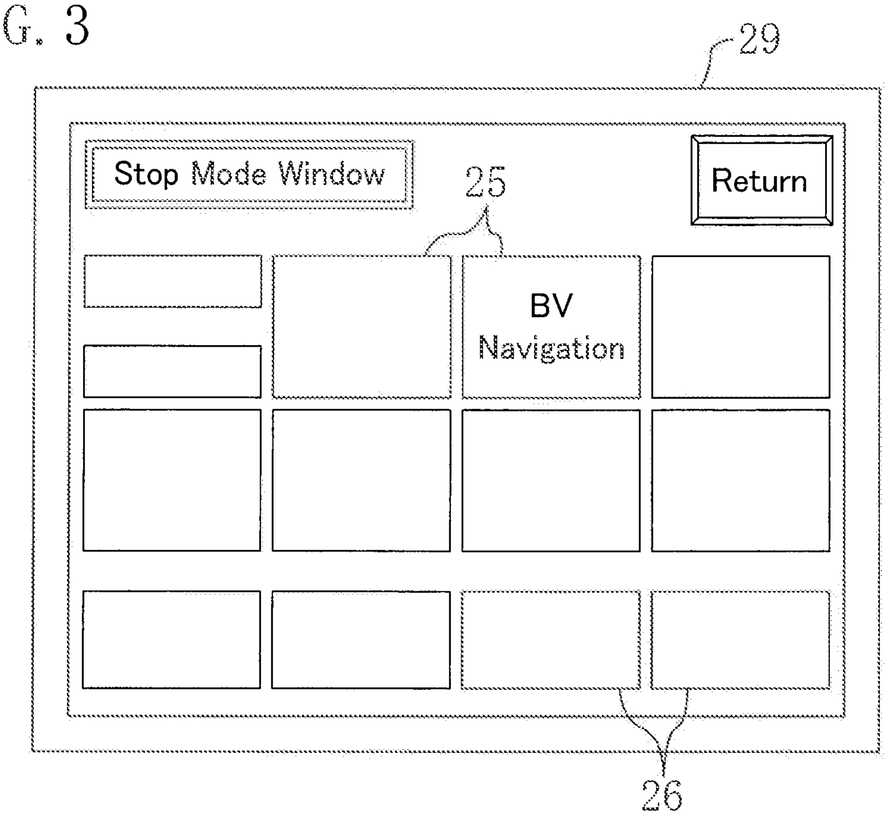
29
Stop Mode Window
25
Return
BV
Navigation
26

FIG. 4

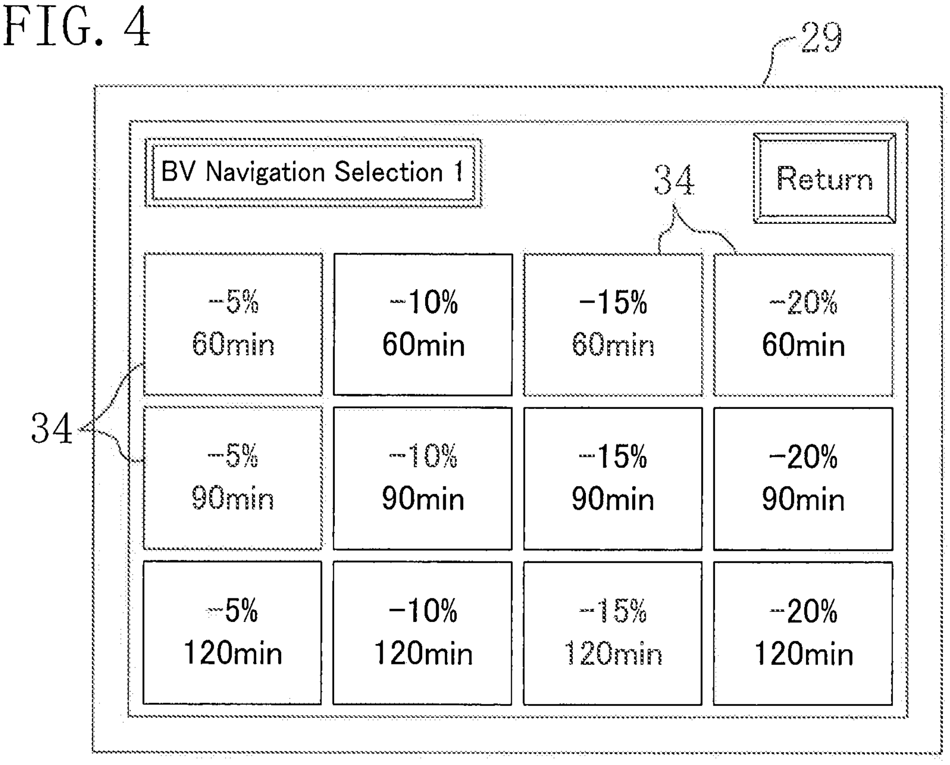
29
BV Navigation Selection 1
34
Return
-5%
60min
-10%
60min
-15%
60min
-20%
60min
34
-5%
90min
-10%
90min
-15%
90min
-20%
90min
-5%
120min
-10%
120min
-15%
120min
-20%
120min

FIG. 5

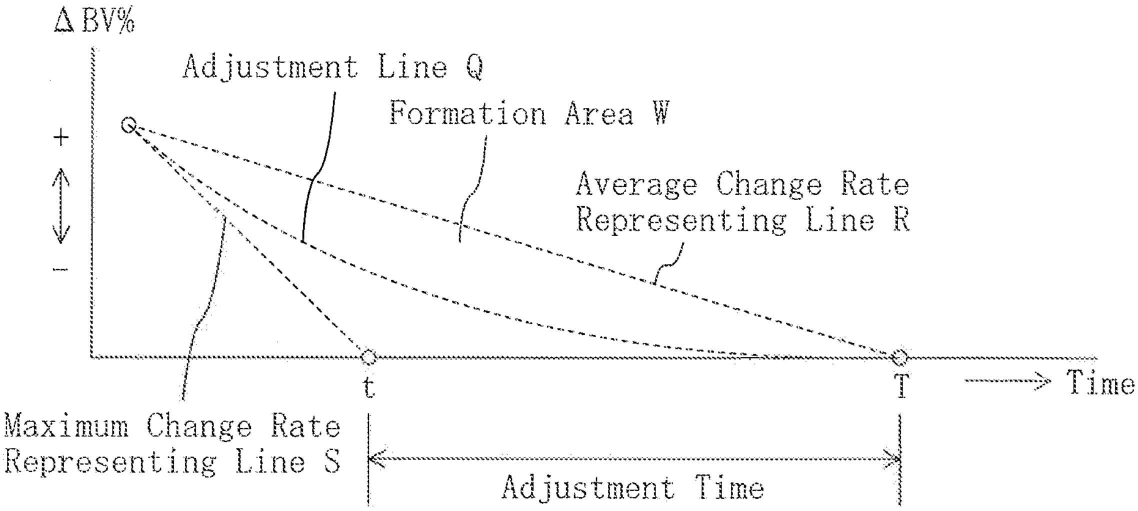
ΔBV%
Adjustment Line Q
Formation Area W
Average Change Rate
Representing Line R
+
−
t
T
Time
Maximum Change Rate
Representing Line S
Adjustment Time

FIG. 6

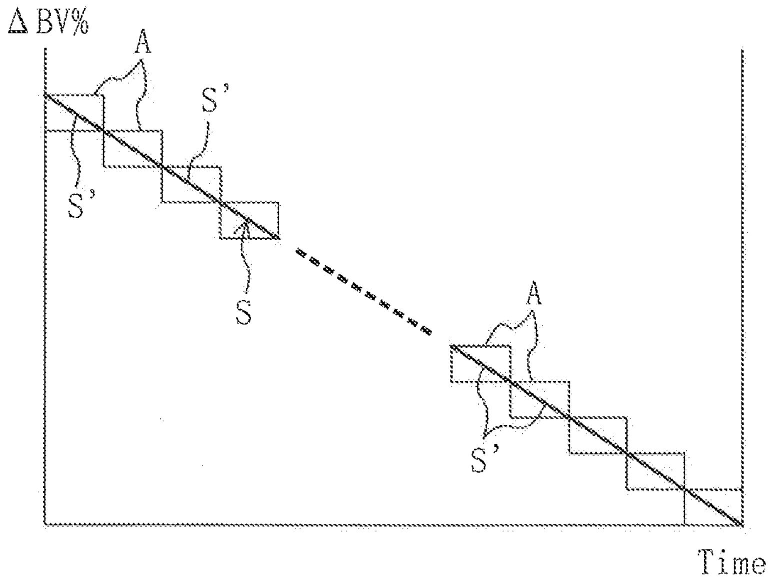
ΔBV%
A
S'
S'
S
A
S'
Time

FIG. 7

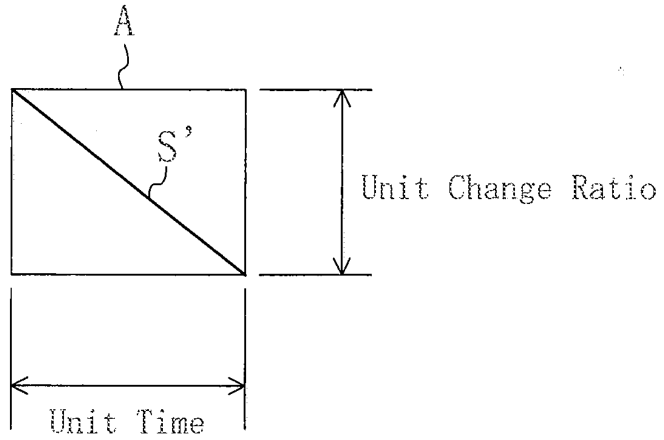
A
S'
Unit Change Ratio
Unit Time

FIG. 8

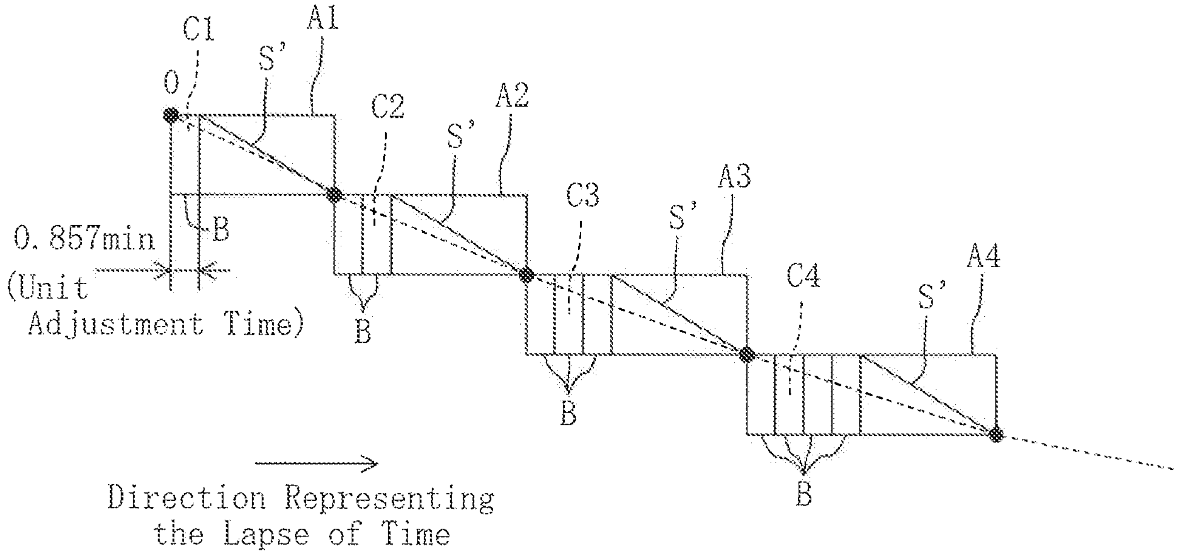
C1
A1
S'
0
C2
A2
S'
C3
A3
S'
B
0.857min
(Unit
Adjustment Time)
B
B
C4
A4
S'
B
Direction Representing
the Lapse of Time

FIG. 9

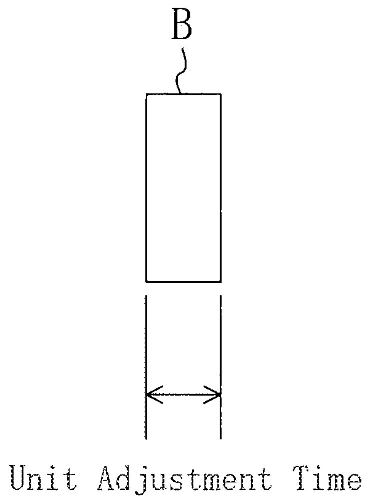
B
Unit Adjustment Time

FIG. 10

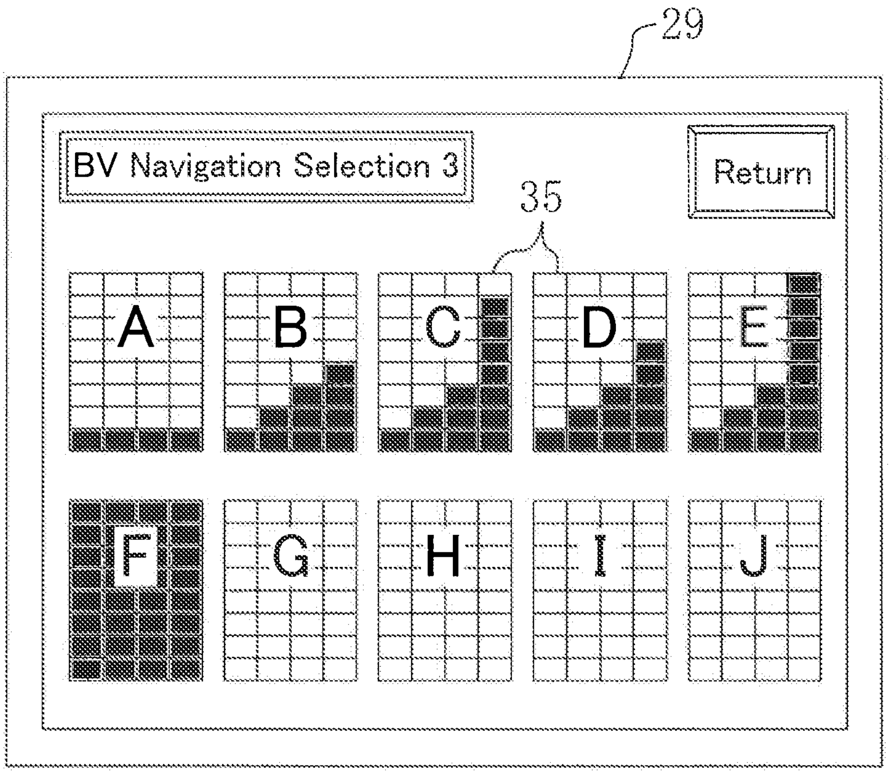
29
BV Navigation Selection 3
Return
35
A
B
C
D
E
F
G
H
I
J

FIG. 11

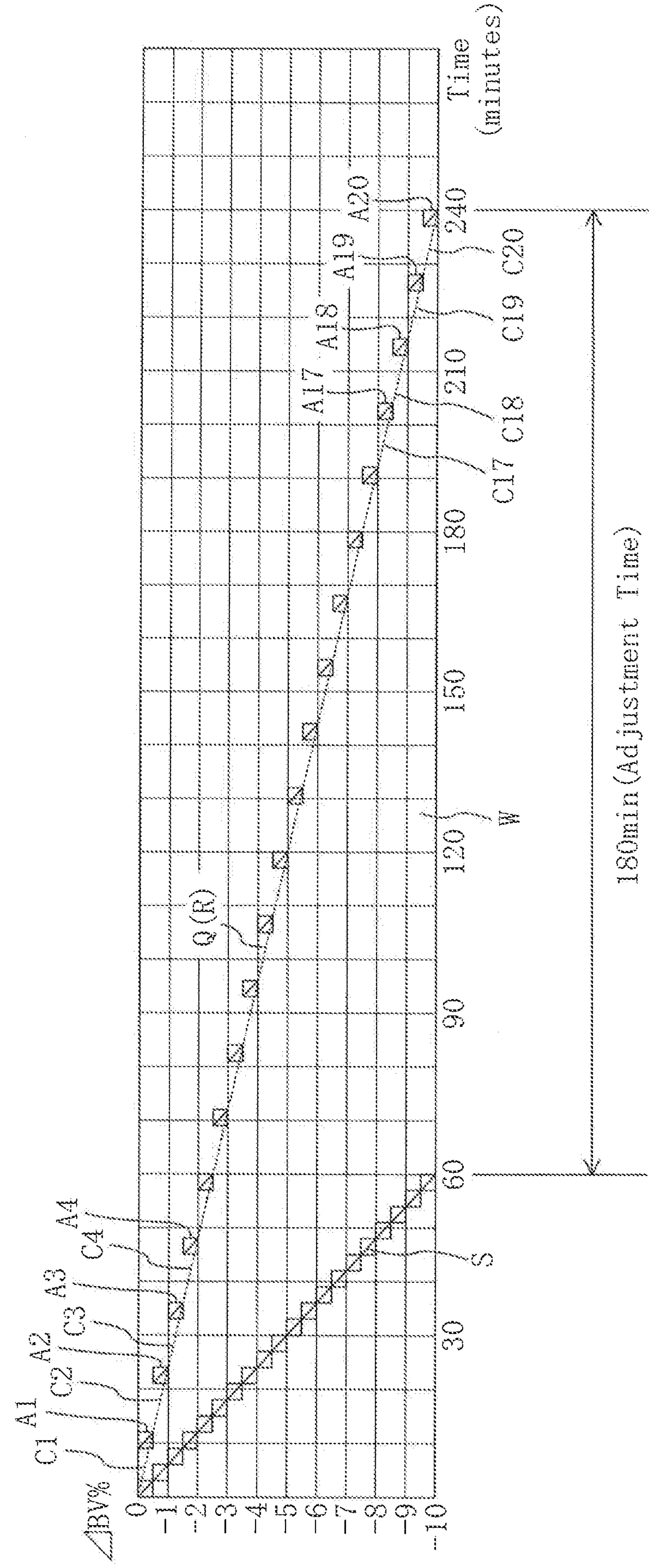

⊿BV%
0
-1
-2
-3
-4
-5
-6
-7
-8
-9
-10
Time (minutes)
30
60
90
120
150
180
210
240
180min (Adjustment Time)
C1
A1
C2
A2
C3
A3
C4
A4
Q(R)
W
S
A17
A18
A19
A20
C17
C18
C19
C20

FIG. 12

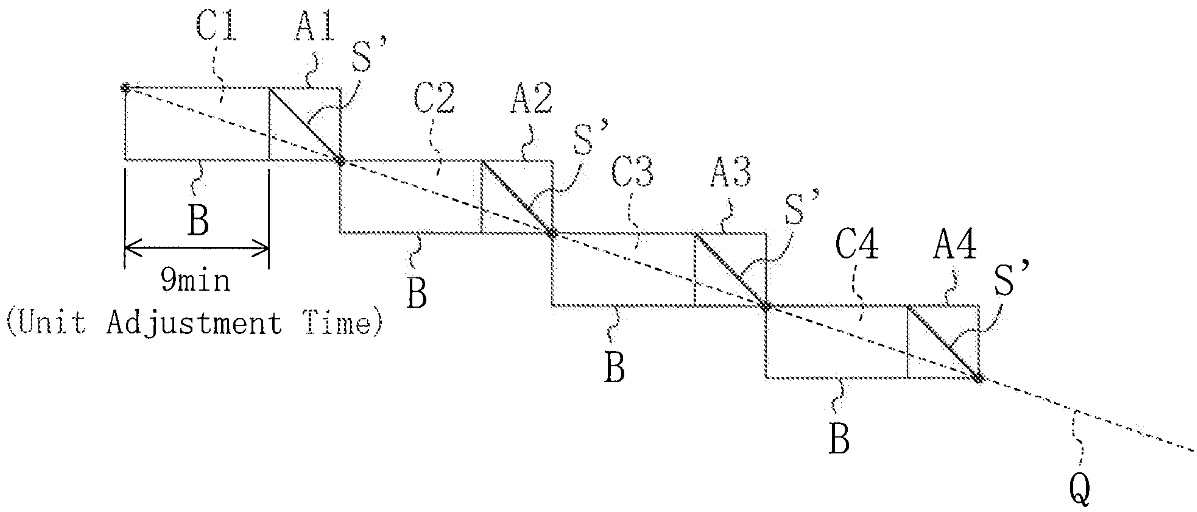

FIG. 13

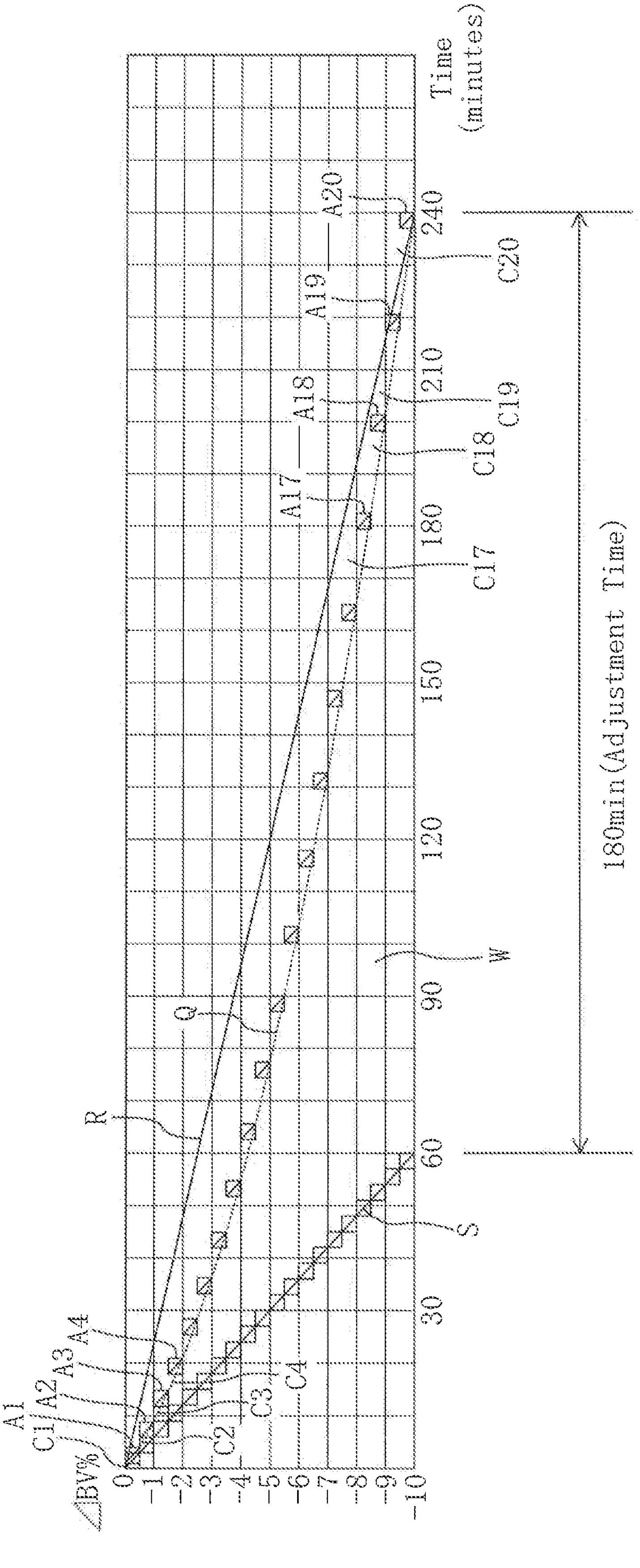

ΔBV%
0
-1
-2
-3
-4
-5
-6
-7
-8
-9
-10
A1
C1
A2
A3
A4
C2
C3
C4
R
Q
S
W
A17
A18
A19
A20
C17
C18
C19
C20
30
60
90
120
150
180
210
240
Time (minutes)
180min(Adjustment Time)

FIG. 14

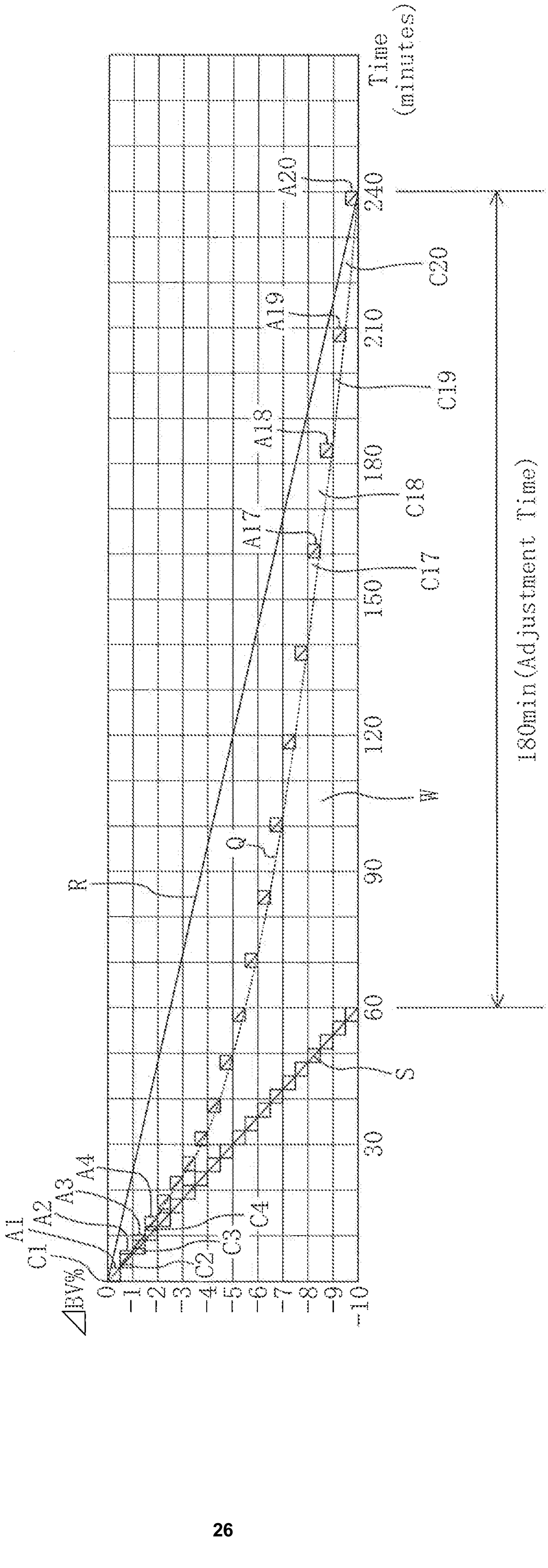
Time (minutes)
ΔBV%
0
-1
-2
-3
-4
-5
-6
-7
-8
-9
-10
30
60
90
120
150
180
210
240
A1
A2
A3
A4
C1
C2
C3
C4
A17
A18
A19
A20
C17
C18
C19
C20
R
Q
W
S
180min(Adjustment Time)

FIG. 15

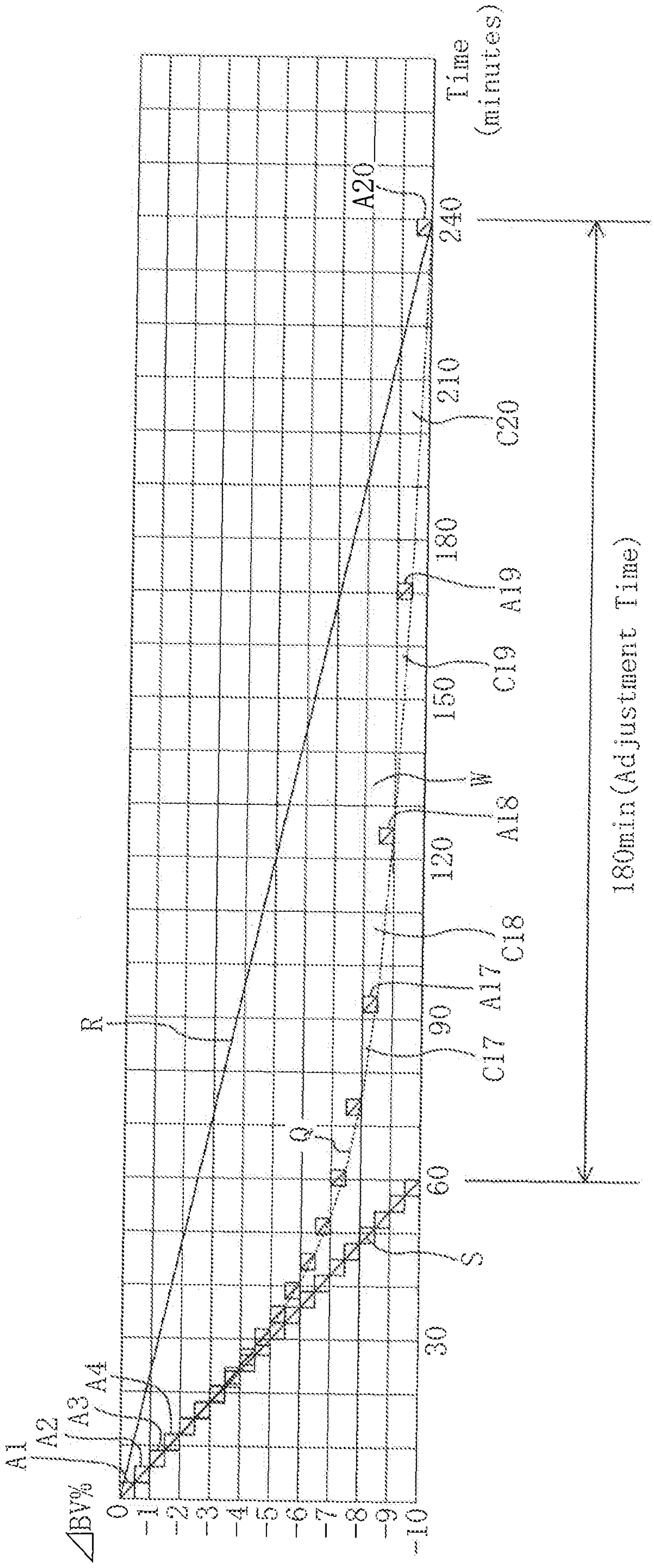

Time (minutes)
ΔBV%
0
-1
-2
-3
-4
-5
-6
-7
-8
-9
-10
30
60
90
120
150
180
210
240
A1
A2
A3
A4
S
Q
R
C17
A17
C18
A18
W
C19
A19
C20
A20
180min (Adjustment Time)

FIG. 16

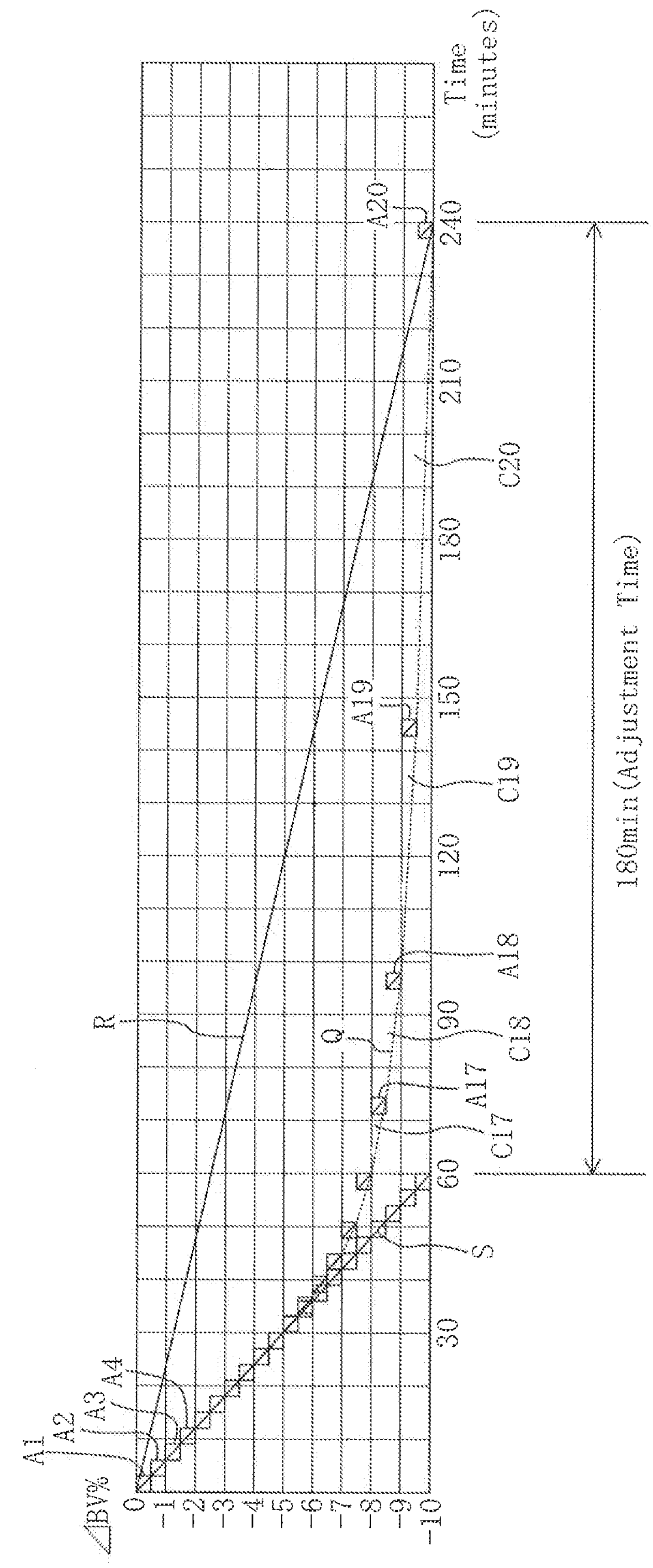

⊿BV%
0
-1
-2
-3
-4
-5
-6
-7
-8
-9
-10
30
60
90
120
150
180
210
240
Time (minutes)
A1
A2
A3
A4
A17
A18
A19
A20
C17
C18
C19
C20
R
Q
S
180min (Adjustment Time)

FIG. 17

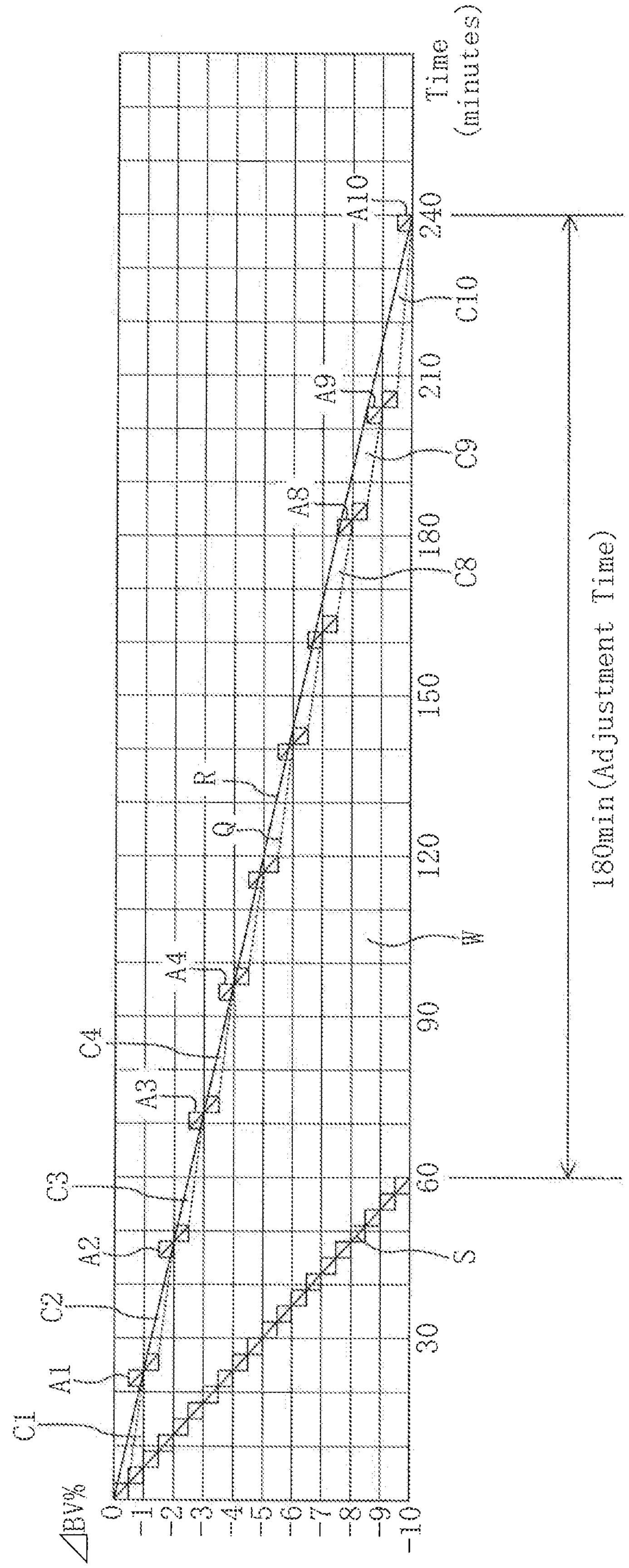

ΔBV%
0
-1
-2
-3
-4
-5
-6
-7
-8
-9
-10
30
60
90
120
150
180
210
240
Time (minutes)
C1
A1
C2
A2
C3
A3
C4
A4
Q
R
W
S
A8
C8
A9
C9
C10
A10
180min(Adjustment Time)

FIG. 18

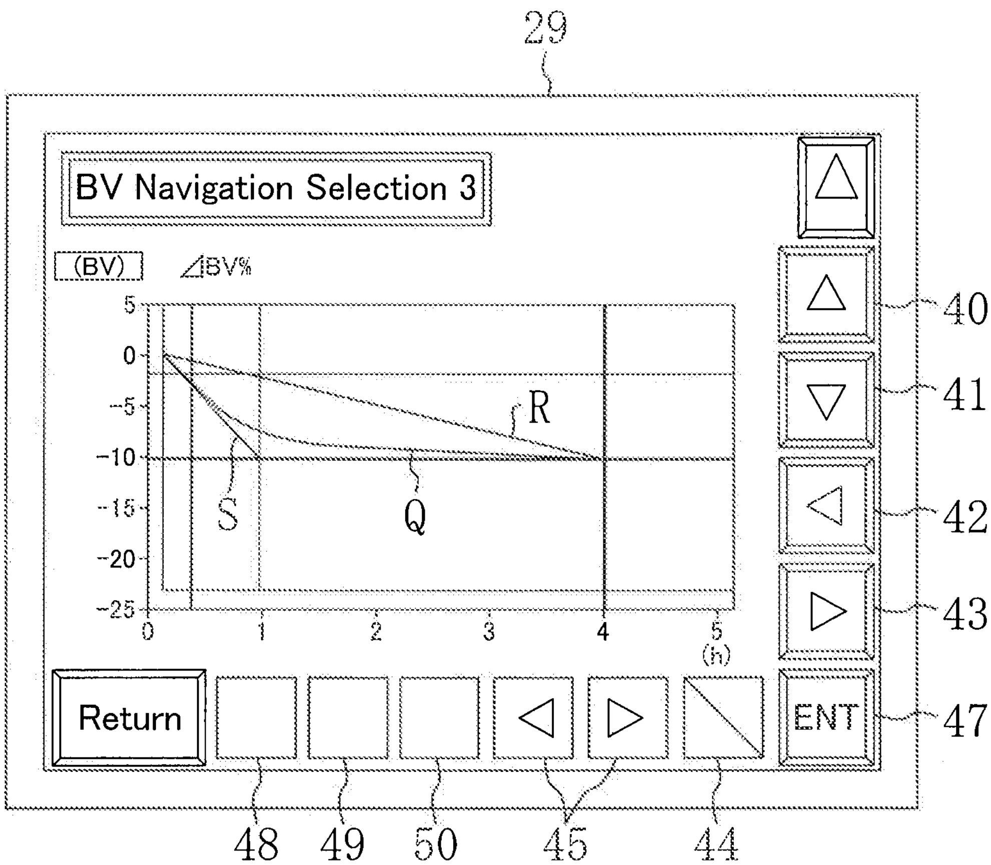
29
BV Navigation Selection 3
(BV)
⊿BV%
5
0
-5
-10
-15
-20
-25
0
1
2
3
4
5
(h)
R
S
Q
40
41
42
43
ENT
47
Return
48
49
50
45
44

FIG. 19

(a)

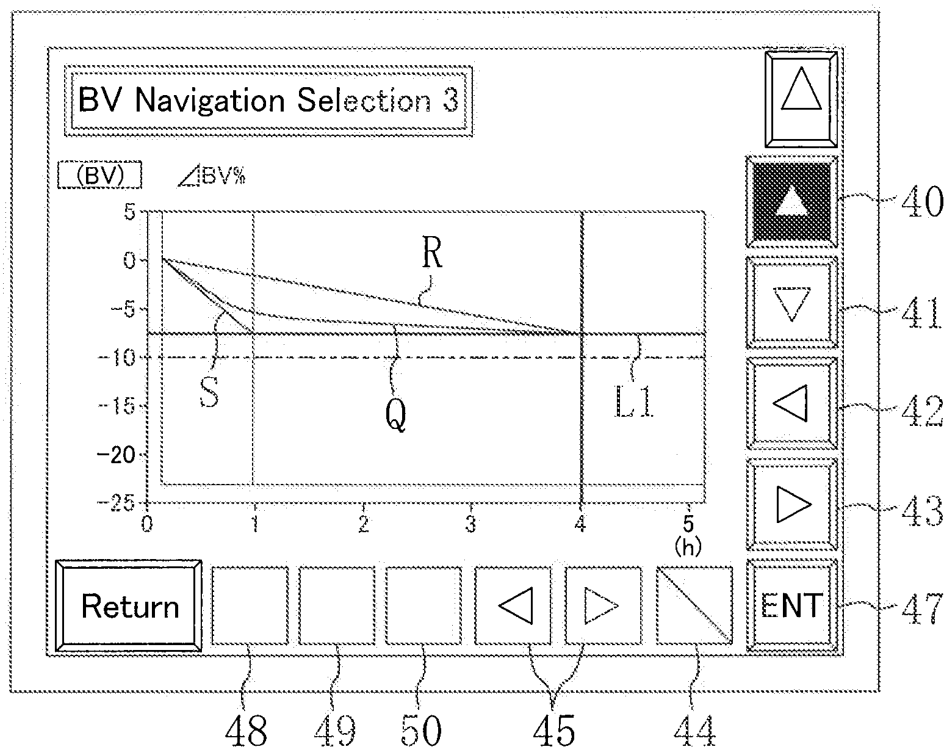

BV Navigation Selection 3
(BV)
⊿BV%
5
0
-5
-10
-15
-20
-25
0
1
2
3
4
5
(h)
R
S
Q
L1
Return
ENT
40
41
42
43
47
48
49
50
45
44


(b)

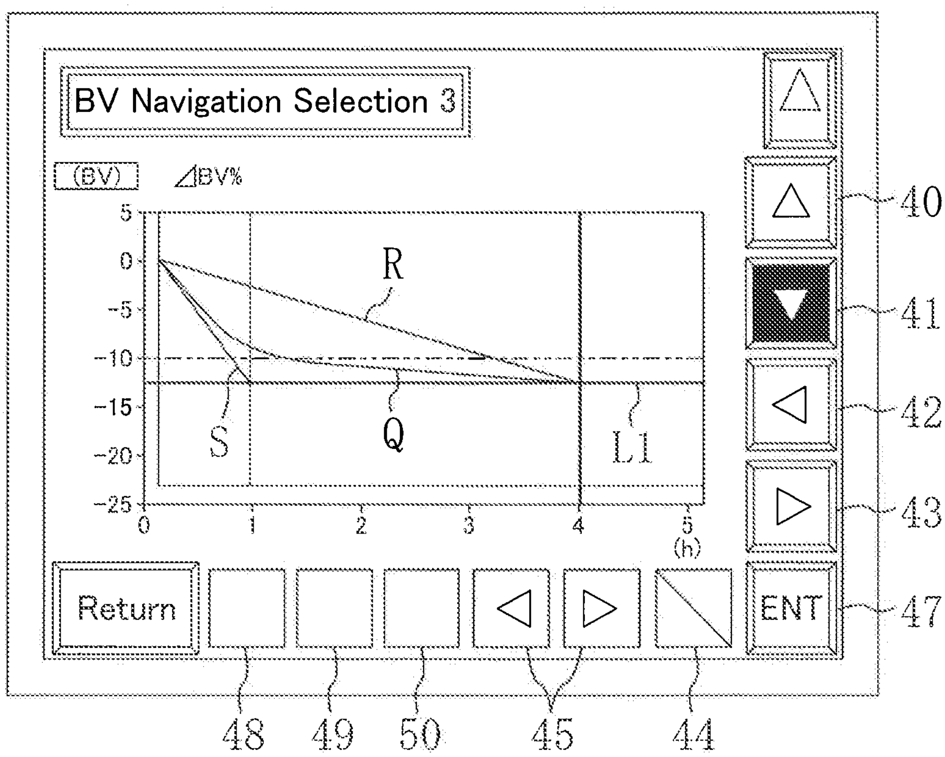

BV Navigation Selection 3
(BV)
⊿BV%
5
0
-5
-10
-15
-20
-25
0
1
2
3
4
5
(h)
R
S
Q
L1
Return
ENT
40
41
42
43
47
48
49
50
45
44

FIG. 20

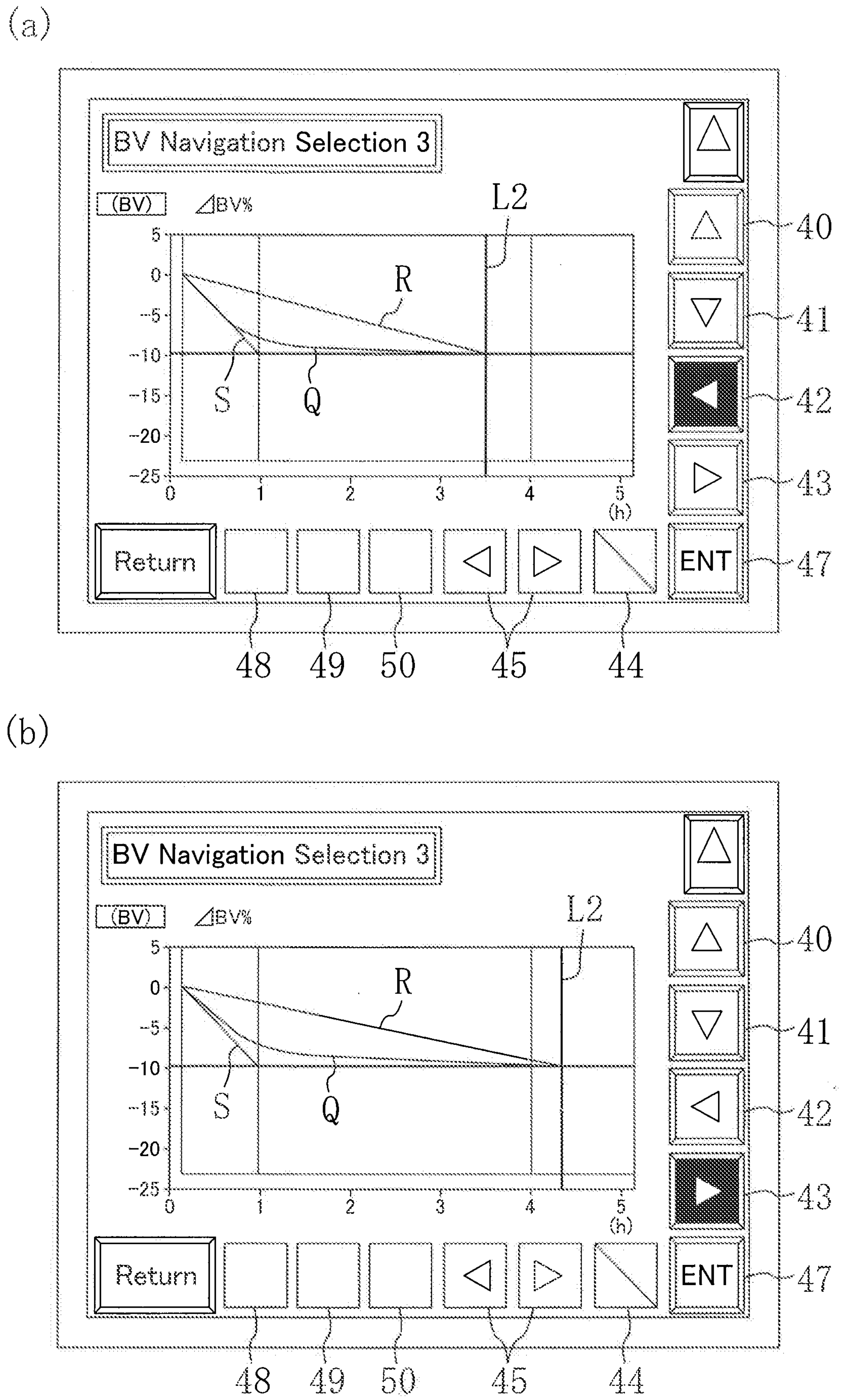
(a)
BV Navigation Selection 3
(BV)
⊿BV%
L2
R
S
Q
5
0
-5
-10
-15
-20
-25
0
1
2
3
4
5
(h)
Return
ENT
40
41
42
43
47
48
49
50
45
44
(b)
BV Navigation Selection 3
(BV)
⊿BV%
L2
R
S
Q
Return
ENT
40
41
42
43
47
48
49
50
45
44

FIG. 21

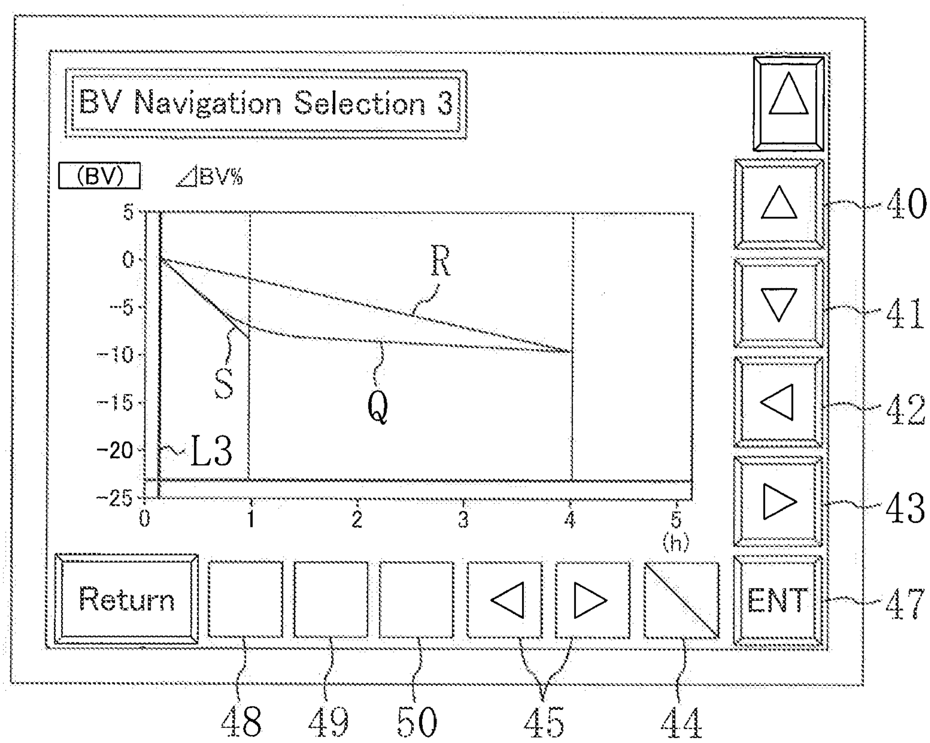
BV Navigation Selection 3
(BV)
⊿BV%
5
0
-5
-10
-15
-20
-25
0
1
2
3
4
5
(h)
R
S
Q
L3
Return
ENT
40
41
42
43
47
48
49
50
45
44

FIG. 22

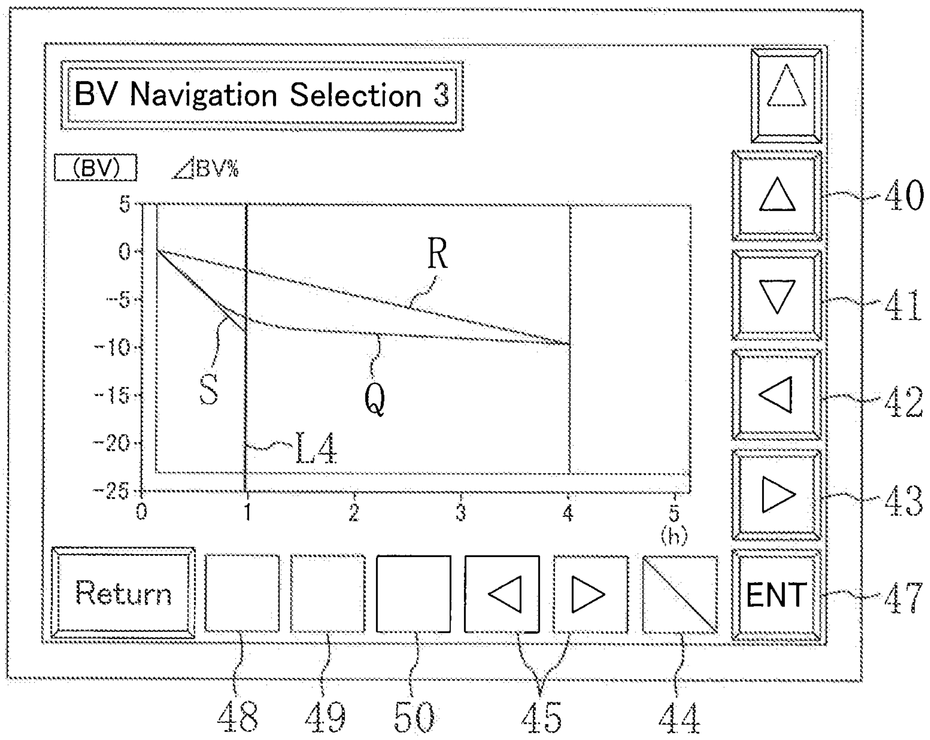
BV Navigation Selection 3
(BV)
⊿BV%
5
0
-5
-10
-15
-20
-25
0
1
2
3
4
5
(h)
R
S
Q
L4
Return
ENT
40
41
42
43
47
48
49
50
45
44

FIG. 23

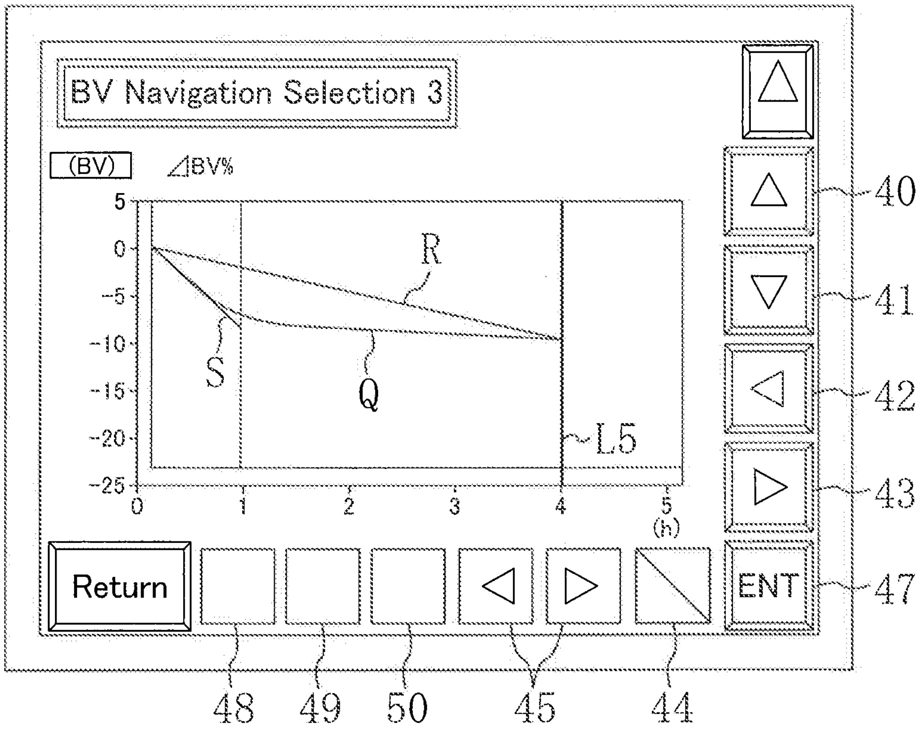
BV Navigation Selection 3
(BV)
⊿BV%
5
0
-5
-10
-15
-20
-25
0
1
2
3
4
5
(h)
R
S
Q
L5
Return
ENT
40
41
42
43
47
48
49
50
45
44

FIG. 24

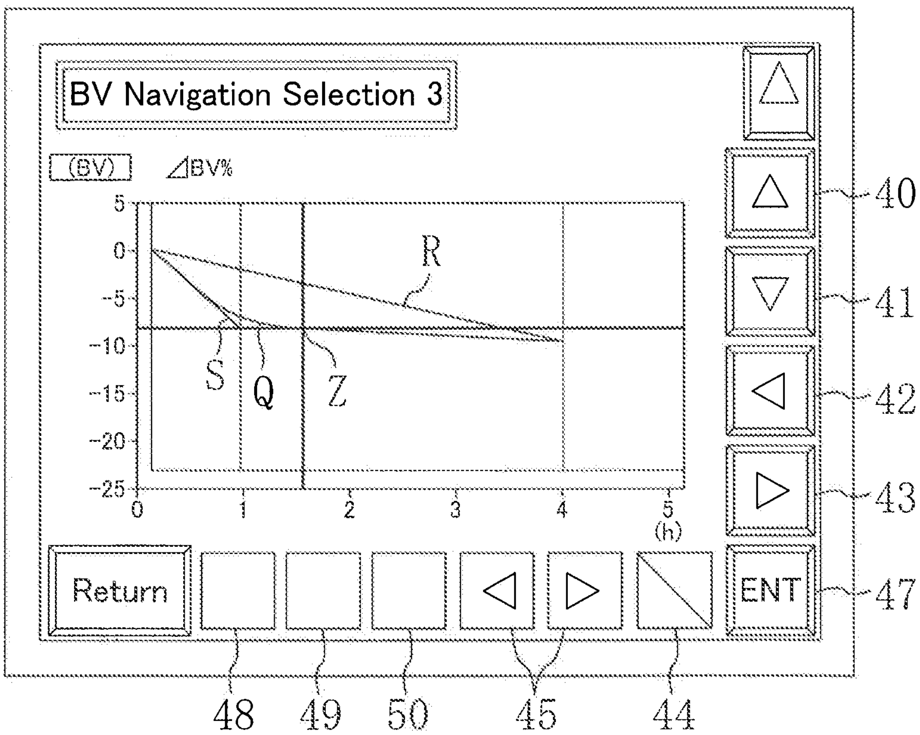
BV Navigation Selection 3
(BV)
⊿BV%
5
0
-5
-10
-15
-20
-25
0
1
2
3
4
5
(h)
R
S
Q
Z
Return
ENT
40
41
42
43
47
48
49
50
45
44

FIG. 25

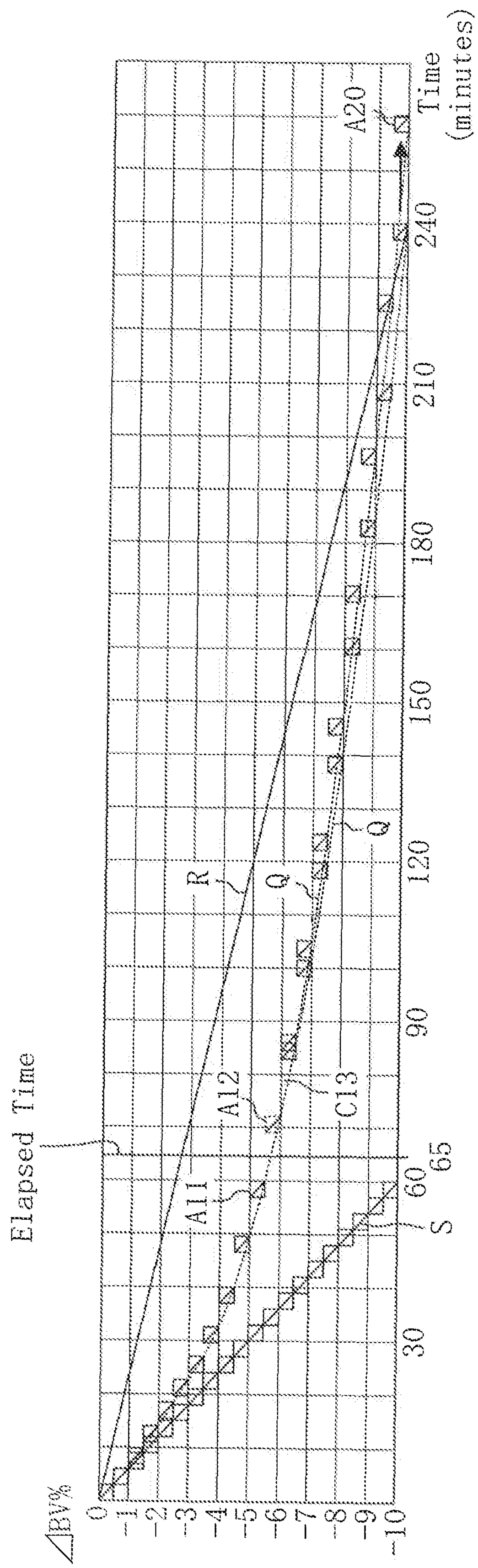
ΔBV%
0
-1
-2
-3
-4
-5
-6
-7
-8
-9
-10
30
60
65
90
120
150
180
210
240
Time (minutes)
Elapsed Time
A11
A12
C13
R
Q
Q
S
A20

FIG. 26

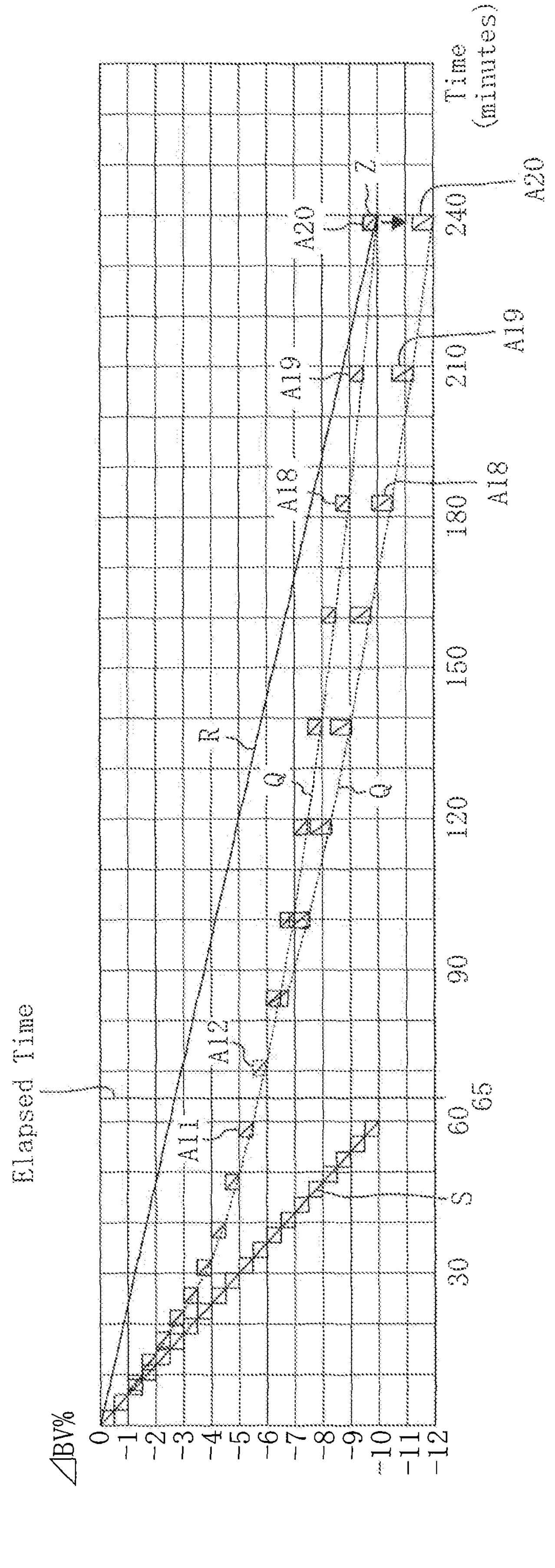

△BV%
0
-1
-2
-3
-4
-5
-6
-7
-8
-9
-10
-11
-12
30
60
65
90
120
150
180
210
240
Time
(minutes)
Elapsed Time
A11
A12
R
Q
Q
A18
A18
A19
A19
A20
A20
Z
S

FIG. 27

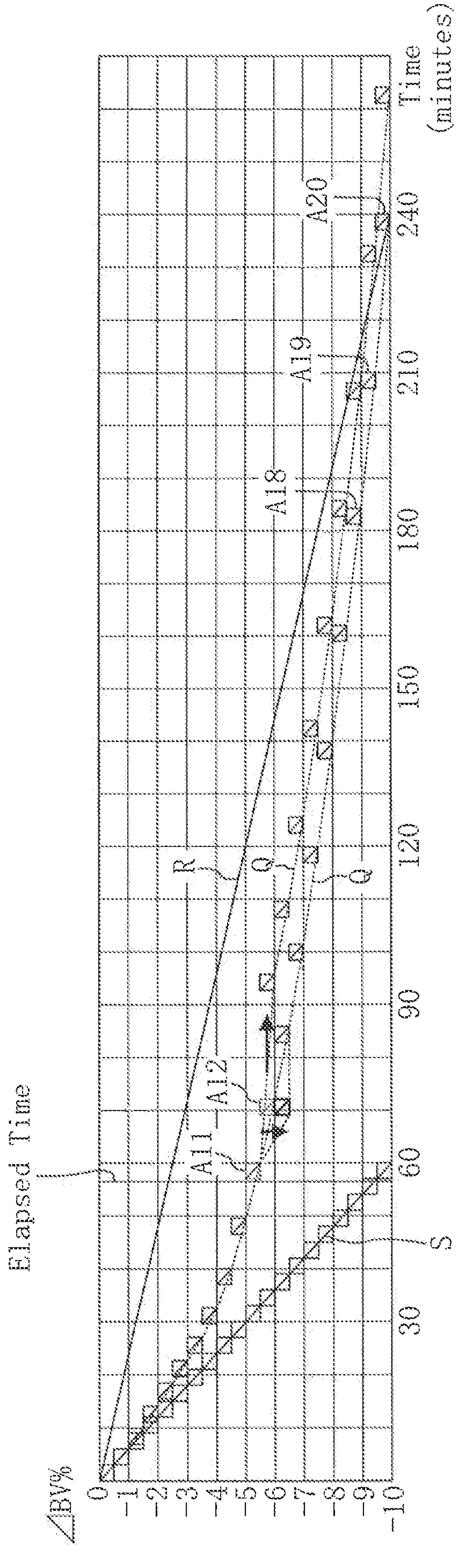
∠BV%
0
-1
-2
-3
-4
-5
-6
-7
-8
-9
-10
30
60
90
120
150
180
210
240
Time
(minutes)
Elapsed Time
A11
A12
A18
A19
A20
R
Q
Q
S

**INTERNATIONAL SEARCH REPORT**

International application No.
PCT/JP2009/000480

A. CLASSIFICATION OF SUBJECT MATTER
*A61M1/14*(2006.01)i

According to International Patent Classification (IPC) or to both national classification and IPC

B. FIELDS SEARCHED

Minimum documentation searched (classification system followed by classification symbols)
A61M1/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y<br><br>A | JP 2004-97781 A (Nikkiso Co., Ltd.),<br>02 April, 2004 (02.04.04),<br>Abstract; Par. Nos. [0071], [0072]<br>& US 2004/0057037 A1 & EP 1543852 A1<br>& WO 2004/009155 A1 | 1-4,7-14,<br>16-20<br>5,6,15 |
| Y<br><br>A | WO 2003/004076 A1 (JMS Co., Ltd.),<br>16 January, 2003 (16.01.03),<br>Claim 1<br>& US 2004/0168988 A1 & EP 1413324 A1 | 1-4,7-14,<br>16-20<br>5,6,15 |
| Y<br><br>A | WO 2003/009888 A1 (JMS Co., Ltd.),<br>06 February, 2003 (06.02.03),<br>Claim 1<br>& US 2004/0238418 A1 & EP 1419794 A1 | 1-4,7-14,<br>16-20<br>5,6,15 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 February, 2009 (24.02.09) | 10 March, 2009 (10.03.09) |
| Name and mailing address of the ISA/<br>Japanese Patent Office | Authorized officer |
| Facsimile No. | Telephone No. |

Form PCT/ISA/210 (second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2007130300 A